# EUROPÄISCHE PATENTSCHRIFT

(11) **EP 1 337 322 B1**
(45) Veröffentlichungstag und Bekanntmachung des Hinweises auf die Patenterteilung: **09.06.2004**
(21) Anmeldenummer: 01992612.0
(22) Anmeldetag: 31.10.2001
(51) Int. Cl.: B01J 13/02, A61K 7/00, A61K 9/127

(54) **VERFAHREN UND VORRICHTUNG ZUR HERSTELLUNG VON LIPIDVESIKELN**
METHOD AND DEVICE FOR PRODUCING LIPID VESICLES
PROCEDE ET DISPOSITIF POUR PRODUIRE DES VESICULES LIPIDIQUES

(30) Priorität: 03.11.2000 EP 00123953
(43) Veröffentlichungstag der Anmeldung: 27.08.2003
(73) Patentinhaber: Polymun Scientific Immunbiologische Forschung GmbH, 1190 Wien (AT)
(72) Erfinder: KATINGER, Hermann, A-1190 Wien (AT); VORAUER-UHL, Karola, A-1220 Wien (AT); WAGNER, Andreas, A-2500 Baden (AT); KREISMAYR, Günter, A-1170 Wien (AT)
(74) Vertreter: Büchel, Kurt F., Dr.
(86) Internationale Anmeldenummer: PCT/EP2001/012595
(87) Internationale Veröffentlichungsnummer: WO 2002/036257

(56) Entgegenhaltungen:
- WO-A-00/29103
- US-A- 4 895 452
- US-A- 5 000 887

## Beschreibung

Die Erfindung betrifft ein Verfahren und eine Vorrichtung zur besonders schonenden Herstellung von Vesikeln, insbesondere Lipidvesikeln, im Laborund Industriemassstab.

### STAND DER TECHNIK

Im Stand der Technik sind zahlreiche unterschiedliche Vesikelpräparationen bekannt. Sie umfassen sowohl reine Lipidvesikel, auch Liposomen bezeichnet, als auch Lipidvesiket, die mit zell-spezifischen Markern wie z.B. Antikörpern oder Antikörperfragmenten, insbesondere Fab' und F(ab')₂, bestückt sind und meist als Immunoliposomen bezeichnet werden, oder Lipidvesikel, die mit viralen Hüllproteinen oder Oberflächenantigenen, beispielsweise Hämagglutinin, bestückt sind und üblicherweise als Virosomen bezeichnet werden. Darüber hinaus sind auch Lipidvesikel bekannt geworden, welche sowohl virale Antigene als auch spezifische Markermoleküle in der Vesikelmembran enthalten. Die Vesikel enthalten im Allgemeinen eine wässrige Phase, die im einfachsten Fall reines Wasser, wässrige Puffersysteme, wie z.B. PBS, oder eine physiologische Kochsalzlösung sein kann. Für pharmazeutische und medizinische Anwendungen einschliesslich diagnostischer Anwendungen enthalten die Vesikel jedoch zumeist eine oder mehrere gewünschte Substanzen, beispielsweise einen physiologisch aktiven Wirkstoff, einen Arzneistoff, einen speziellen Farbstoff oder auch Nukleinsäurematerial.

Die einfachste und am weitesten verbreitete Kategorie von Lipidvesikeln sind die Liposomen. Sie sind zumeist aus Phospholipiden aufgebaut, die sich spontan zu Membranstrukturen formieren, wenn sie in wässrigen Systemen dispergiert werden. Dabei wird ein Teil des wässrigen Systems von diesen Strukturen im Zuge der Vesikelbildung eingeschlossen. In der Praxis sind meist solche Liposomen bevorzugt, deren Vesikelmembran wie natürliche Membranen als Lipid-Doppelschicht ("bi-layer") ausgebildet ist.

Seit der erstmaligen Präsentation von Liposomen durch Bangham 1964 (J.Mol.Biol. 13:238-252, 1965) wurden eine Reihe von verschiedenen Liposomen-Präparationstechniken etabliert. Dazu gehören Verfahren, bei denen Lipide zusammen mit dem gewünschten Wirkstoff in einem geeigneten wässrigen Lösungsmittel vermischt und anschliessend mit Ultraschall beschallt werden, wodurch sich Vesikel formieren. In anderen Verfahren, beispielsweise nach Stegman et al. (EMBO Journal 6:2651-2659, 1987) wird das Lösungsmittel durch Dialyse oder mittels Microcarriern langsam entfernt, wobei sich ebenfalls spontan Vesikel ausbilden, die einen Teil der wässrigen Phase einschliessen.

Bekannte Hochdruckhomogenisations-, Microfluidizer- und Ultraschallverfahren zur Herstellung von Liposomen zeichnen sich durch Nutzung der im Prozess entstehenden Scherkräfte und Kavitationseffekte aus, um Lipidvesikel definierter Grösse zu erzielen. Kavitationseffekte führen lokal zu sehr hohen Drücken und Temperaturen. Liposomen, insbesondere ungesättigte Fettsäurereste der Lipide, können dadurch oxidativ geschädigt werden und damit ihre Stabilität verändern. Aus diesem Grund müssen solchen Verfahren Antioxidanzien oder andere schützende Ingredienzien zugesetzt und/oder das ganze Verfahren unter Ausschluss von Sauerstoff, beispielsweise in einer Stickstoffatmosphäre, durchgeführt werden, wie z.B. in EP 0 253 619 oder US 5,834,016 beschrieben. Die Herstellung der Lipidvesikel erfolgt in diesen Verfahren in mehreren Zyklen.

Weiters kann man durch sogenannte "Extrusion" grosse Volumina von Liposomen mit definierter Grösse herstellen. Bei der Extrusion werden vorgefertigte Lipidmembranstrukturen, beispielsweise im Handel erhältliche. grosse ( > 500 nm) Liposomen, mit hohem Druck durch Membranen mit definierter Porengrösse gepresst und so auf den gewünschten Durchmesser eingestellt. Dabei müssen, ähnlich wie bei Hochdruckhomogenisation und Ultraschallmethoden mehrere Zyklen durchlaufen werden, um eine enge Grössenverteilung des Endprodukts zu erhalten. Ein möglicher Nachteil dieser Methode besteht in erheblichen Produktverlusten, die durch Aufbrechen und Wiederverschliessen der Vesikel im Zuge der Passage durch die Membranen entstehen. Produktverluste von weit über 50 Prozent können bei diesem Verfahren durchaus vorkommen, wie eigene Vergleichsversuche gezeigt haben.

Eine andere Methode zur Herstellung von Lipidvesikeln einheitlicher Grvssenverteifung wird in US 5,000,887 beschrieben. Dabei werden Lipide in einem mit Wasser mischbaren Lösungsmittel, z.B. Ethanol, gelöst und wässriges Medium hinzugefügt, bis ein Mischungsverhältnis erreicht wird, bei dem Vesikelbildung einsetzt. Anschliessend wird das Verhältnis von Wasser zu Lösungsmittel unter Konstanthaltung des Mischungsvolumens solange angehoben, bis einheitlich grosse Vesikel entstehen, wobei die Durchschnittsgrösse der Vesikel durch Variation der lonenstärke und die Auswahl der Lipidzusammensetzung einstellbar ist.

Eine andere Methode, die sich der spontanen Vesikelbildung beim Phasenübergang von organischer zu anorganischer Phase bedient, ist die Lösungsmitttelinjektionsmethode. Der "self-assembly"-Prozess (spontane Anordnung der Lipide zu Vesikeln) findet während der mit einer feinen Nadel durchgeführten Injektion der in einer organischen Phase gelösten Lipide in eine kräftig gerührte wässrige (oder andere geeignete polare) Phase statt. Diese Methode ist sehr einfach, hat jedoch den grossen Nachteil, dass bei höheren Lipidkonzentrationen sehr inhomogene und grosse Liposomen produziert werden. Diese Methode wurde erstmals von Batzri und Korn (Biochimica et Biophysica Acta, 298:1015-1019, 1973) beschrieben.

EP 253 619 beschreibt ein Verfahren nach der zuletzt genannten Lösungsmittelinjektionemethode, bei dem eine organische Phase mit gelösten Lipiden unter Druck in einen Hochgeschwindigkeitshetnogenisator lnjiziert und darin durch heftiges Rühren unter starken Turbulenzen in einer wässrigen Phase dispergiert wird, worauf Liposomen gebildet werden, Als möglicher Nachteil dieser Methode ist zu sehen, dass im Homogenisator starke Scherkräfte, Kavitation und örtlich-temporär ganz beträchtliche Temperaturerhöhungen erzeugt werden. Durch derartige Effekte können Oxidationsreaktionen an ungesättigten Fettsäureresten sowie andere unerwünschte Reaktionen ausgelöst werden, die sowohl Lipide als auch scherkraft-, oxidations- oder temperaturempfindliche Substanzen oder Wirkstoffe zumindest schädigen. unter Umständen sogar für die weitere Verwendung unbrauchbar machen können.

Ein ähnliches Verfahren wird auch in US 4,895,452 beschrieben, bei weichem multi- oder paucilamellare Vesikel in einer eigenen Mischkammer erzeugt werden, in welche die Flüssigkeitsströme der polaren und der lipidhältigen Phasen tangential injiziert werden, um auf diese Weise möglichst hohe Scherkräfte und Durchmischung zu erzielen.

Auch WO 00/29103 offenbart ein Verfahren, bei dem Lipidvesikel aus einem lipidhältigen und einem wässrigen Flüssigkeitsstrom mittels geeigneter Mischeinrichtungen und unter Einsatz hoher Scherkräfte erzeugt werden.

Die vorliegende Erfindung hingegen, welche im Wesentlichen eine Weiterentwicklung der Lösungsmittelinjektionsmethode darstellt, weist die Nachteile der bekannten Verfahren nicht mehr auf, sondern erlaubt bei extrem schonender Herstellungsweise das gezielte Einstellen einer engen Grössenverteilung von Liposomen einer gewünschten Grösse. Darüber hinaus ermöglicht das erfindungsgemässe Verfahren einen kontinuierlichen Betrieb sowie eine einfache Massstabsvergrösserung für die industrielle Produktion keimfreier und/oder pyrogenfreier Lipidvesikelpräparationen, aber auch eine Massstabsverkleinerung für die Mikroproduktion, d.h. die Produktion von Kleinstmengen von Lipidvesikeln, beispielsweise zum Zwecke der wissenschaftlichen Forschung.

### KURZE BESCHREIBUNG DER ERFINDUNG

Aufgabe der Erfindung ist es, ein im Produktionsmassstab verkleineroder vergrösserbares, insbesondere kontinuierliches, Verfahren zur besonders schonenden Herstellung von Lipidvesikeln bereitzustellen, welches reproduzierbar homogen verteilte Vesikelpräparationen liefert und in einer bevorzugten Ausführungsform die Erzeugung keimfreier und/oder pyrogenfreier Vesikelpräparationen gestattet.

Anders als bei der bekannten Ethanol-Injektionsmethode wird die polare, im Regelfall wässrige, Phase gemäss der vorliegenden Erfindung aus einem Vorratsbehälter in ein daran anschliessendes Leitungssystem mit einer oder mehreren Leitungen gepumpt. Jede von der polaren Phase durchströmte, vom Vorratsbehälter wegführende Leitung enthält an einer vorbestimmten Stelle mindestens eine, gegebenenfalls mehrere, sehr exakt ausgeführte, seitlich angeordnete Bohrungen bzw. Öffnungen, die durch die Leitungswand hindurchreichen und an der Aussenseite mit mindestens einer, gegebenenfalls mehreren, Zufuhrleitungen zur druckgesteuerten Einspeisung der in einem geeigneten Lösungsmittel, insbesondere Ethanol, gelösten Lipidphase verbunden ist bzw. sind.

Im Gegensatz zur klassischen Ethanol-Injektionsmethode gibt es bei der erfindungsgemässen Vorrichtung jedoch keine Injektionsnadel, welche in die wässrige Phase hineinreicht, sondern die mindestens eine Zufuhrleitung für die Lipidphase endet erfindungsgemäss an der mindestens einen Bohrung bzw. Öffnung des vom Vorratsbehälter wegführenden Leitungssystems für die wässrige Phase, sodass keine Turbulenzen oder Scherkräfte erzeugenden Hindernisse oder Barrieren in das Innere der Leitungen hineinragen. Durch die druckgesteuerte Einspeisung der Lipidphase in die, vorzugsweise laminar, vorbeiströmende polare/wässrige Phase bilden sich im Bereich des Aufeinandertreffens der beiden Phasen die gewünschten Lipidstrukturen durch einen kontrolliert steuerbaren "self assembly" Effekt zu Vesikeln bisher nicht erzielbar einheitlicher Grösse und schmaler Grössenverteilung aus.

Erstaunlicherweise und völlig unerwartet hat sich dabei ausserdem gezeigt, dass bei der erfindungsgemässen Methode weder höhere Lipidkonzentrationen noch höhere Ethanolkonzentrationen die Vesikelbildung und die Verteilung der Vesikelgrössen zu beeinflussen scheinen. Dies steht in einem gewissen Widerspruch zur bestehenden Meinung der Fachwelt, dass die Vesikelformation von der Lipidkonzentration, der Ethanolkonzentration, den Strömungsgeschwindigkeiten der wässrigen und der organischen Phase sowie von der Rührgeschwindigkeit der Homogenisiervorrichtung abhängen.

Gemäss der vorliegenden Erfindung werden weder Ultraschall noch Homogenisator zur Vesikelformation oder zur Einstellung der gewünschten Vesikelgrösse benötigt. Auch die aus dem Stand der Technik bekannten Filtrationsverfahren ("Extrusionsverfahren") zur Einstellung einer möglichst einheitlichen Vesikelgrösse können durch die vorliegende Erfindung vorteilhaft ersetzt werden. Die Vesikelgrösse und Grössenverteilung lassen sich erfindungsgemäss nämlich vor allem über den Dosierdruck steuern: je höher der Dosierdruck der zugeführten Lipidphase, desto kleiner die Vesikel und desto schärfer bzw. enger ist die Grössenverteilung der entstandenen Lipidvesikel in der nativen Lipidvesikelpräparation, selbst bei hohen Lipidkonzentrationen.

Mit dem erfindungsgemässen Verfahren lassen sich nicht nur wasserlösliche Wirkstoffe in Liposomen einschliessen, sondern auch solche, die in der organischen Phase vorgelegt werden und zusammen mit den Lipiden in das wässrige System eingebracht werden, wie beispielsweise solche, wie sie aus EP 0 253 619 oder US 5,834,016 bekannt sind. In diesen Fällen kann das wässrige System Wasser, physiologische Kochsalzlösung, PBS oder ein beliebiger, geeigneter Puffer sein. Die in der organischen, weitgehend apolaren, Phase vorgelegten Substanzen werden bei der Ausbildung der Lipidvesikel entweder ganz oder teilweise in die Lipiddoppelmembran integriert oder lagern sich durch lipophile Wechselwirkungen eng an diese an. Es ist auch möglich, in beiden Phasen, d.h. sowohl in der polaren als auch in der organischen, lipidhältigen Phase gewünschte Substanzen oder Wirkstoffe vorzulegen und damit Liposomen zu beladen.

Beim vorliegenden Verfahren kann sowohl eine diskontinuierliche als auch eine kontinuierliche Prozessführung gewählt werden. Bei der diskontinuierlichen Verfahrensweise wird ein vorgegebenes Volumen an polarer Phase im System zirkuliert, d.h. ins Ausgangsgefäss zurückgeführt und neuerlich mit lipidhältiger Phase beladen. Durch die Zufuhr der lipidhältigen Phase wird die polare Lösung sukzessive mit Lipidvesikeln angereichert. Beim kontinuierlichen Prozess wird die polare Phase nicht rezirkuliert sondern sie wird nach erfolgter Zudosierung der lipidhältigen Phase und der spontanen Vesikelbildung als Vesikelsuspension in einem externen Sammelbehälter aufgefangen. Die Vesikelgrössen bzw. Grössenverteilungen beider Vesikelsuspensionen werden durch die unterschiedlichen Verfahrensmodi nicht beeinflusst.

Eine Weiterentwicklung des kontinuierlichen Verfahrens besteht darin, dass einerseits polare Pufferphase im externen Sammelbehälter vorgelegt wird und andererseits die sonst übliche Menge an zugeführtem Lipid (und vorzugsweise auch an organischem Lösungsmittel) je Volumenseinheit vorbeiströmender polarer/wässriger Phase um das Zwei- bis Zehnfache erhöht wird. Dadurch kann die volumetrische Produktausbeute (Menge an liposomal inkorporierter, vorzugsweise wirkstoffhältiger, polarer Phase) proportional zur Menge an zugeführter Lipidphase gesteigert werden. Im Falle, dass die liposomal zu inkorporierenden Substanzen lipophil sind und zusammen mit den Lipiden in der organischen Phase vorgelegt werden, ist eine hundertprozentige oder zumindest annähernd hundertprozentige Ausbeute auch ohne Rezirkulierung erzielbar, d.h. die gesamte oder nahezu die gesamte Menge an vorgelegter gewünschter Substanz, beispielsweise eines Arzneistoffes, ist später in den Lipidvesikeln enthalten.

Die hierin beschriebenen Vesikelgrössen und Grössenverteilungen wurden mittels Flow-Cytometrie, adaptiert nach Vorauer-Uhl et al., Cytometrie 39(2): 166-71 (2000), ermittelt. Bei dieser Methode werden, im Unterschied zur konventionellen statischen oder dynamischen Laser-Lichtstreuung, die Vesikel in einem Kapillarsystem einzeln vermessen (pro Messung 10.000 Vesikel). Durch diese Methode können sowohl homogene als auch heterogene Vesikelpopulationen zuverlässig charakterisiert werden.

Erfindungsgemäss werden die polare Phase und die lipidhältige Phase getrennt voneinander transportiert und einem Phasen-Kreuzungsbereich zugeleitet, der vorzugsweise in Form eines Kreuzstrommoduls ausgebildet ist. Das Einströmen der lipidhältigen Phase in die ebenfalls fliessende, polare Phase erfolgt über zumindest eine seitlich angeordnete Öffnung im Leitungssystem der polaren Phase, vorzugsweise innerhalb des Kreuzstrommoduls. Die beim Aufeinandertreffen der beiden Phasen entstehenden Lipidvesikel werden zusammen mit der polaren (z.B. wässrigen) Phase entweder in den Ausgangsbehälter zurückgeführt oder in einen Sammelbehälter weiterbefördert. Die Formierung der Lipidvesikel erfolgt spontan im Phasenkreuzungsbereich, d.h. im Bereich des Aufeinandertreffens der polaren und der lipidhältigen Phasen. Um unerwünschte Kavitationseffekte und/oder Scherkräfte sowie damit einhergehende örtliche Temperaturerhöhungen weitestgehend zu vermeiden, wird der Flüssigkeitsstrom zumindest der polaren Phase vorzugsweise möglichst laminar der Öffnung bzw. den Öffnungen zugeleitet. Bei besonders empfindlichen Substanzen oder Substanzgemischen kann es von Vorteil sein, auch flussabwärts vom Kreuzungsbereich der Phasen ein scherkraftarmes Ftiessverhalten der entstandenen Phasendispersion anzustreben.

Im Gegensatz zu den bekannten Lipid/Ethanol-Injektionsmethoden wird gemäss vorliegender Erfindung die Flüssigkeit im Kreuzungsbereich der Phasen weder gerührt noch wird ein Homogenisator oder eine sonstige mechanische Rühr- oder Dispergierhilfe eingesetzt. Die Einspeisung der lipidhältigen Phase in die polare/wässrige Phase erfolgt unter relativ niedrigem Druck und somit mit geringer Wirbelbildung, wobei die Lipidphase vermutlich als eine Art Sprühnebel aus der Leitungsöffnung austritt und sehr rasch mit der polaren Phase in Wechselwirkung tritt. Durch Regelung der Zuflussraten und Fliessgeschwindigkeiten sowohl der polaren als auch der lipidhältigen Phase und durch Variation des Dosierdrucks zum Einspeisen bzw. "Einsprühen" der lipidhältigen Phase ist der "self assembly"-Prozess der Lipidvesikel steuerbar. Mit steigendem Dosierdruck werden die entstehenden Lipidvesikel kleiner und gleichzeitig wird deren Grössenverteilung enger.

Mit Hilfe der vorliegenden Erfindung sind Lipidvesikelpräparationen in unterschiedlichen Produktionsmassstäben - von Versuchs- bzw. Laborgrössen bis zu industriellen Dimensionen - herstellbar. Da die Vesikelbildung nicht in einem gerührten Gefäss, sondern in einem Kernstück des Leitungssystems, vorzugsweise in dem erwähnten Kreuzstrommodul, kontinuierlich stattfindet, werden bei erfindungsgemässer Herstellung unter Konstanthaltung der wesentlichen Prozessparameter wie Dosierdruck der lipidhältigen Phase, Strömungsvolumen der polaren Phase, Volumsverhältnis zwischen polarer und lipidhältiger Phase und Lipidkonzentration der lipidhältigen Phase, immer Präparationen von einheitlicher und reproduzierbarer Qualität hinsichtlich Produkteinschlussrate, Vesikelgrösse und Vesikelgrößenverteilung produziert, unabhängig vom gewählten Produktionsmassstab. Mit der Wahl entsprechender Leitungsquerschnitte und/oder einer Erhöhung der Zahl und/oder des Durchmessers der Öffnung(en) im Leitungssystem der polaren Phase kann ausserdem das Durchsatzvolumen flexibel an verschiedene Produktionsvorgaben angepasst werden.

Das Herstellungsprinzip erlaubt es auch, die im Phasenkreuzungsbereich z.B. im Kreuzstrommodul, entstehende Phasendispersion zumindest teilweise im Kreis zu führen und, gegebenenfalls mehrmals hintereinander, erneut dem Phasenkreuzungsbereich zuzuführen und mit lipidhältiger Phase zu beladen.

Die Einschlussrate (Ausnutzungsgrad) der in der polaren Phase gelösten Substanzen in die Lipidvesikel kann auch dadurch gesteigert werden, dass man den Volumensanteil der lipidhältigen Phase erhöht und pro Zeiteinheit grössere Volumina lipidhältiger Phase in den Strom der polaren Lösung einbringt. Stromabwärts vom Phasenkreuzungsbereich kann die entstandene Phasendispersion mit einem polaren Lösungsmittel, beispielsweise mit dem Trägermedium der polaren Phase, verdünnt werden, um - falls erforderlich - die Stabilität der Liposomenpräparation zu erhöhen.

Die vorliegende Erfindung umfasst auch eine Vorrichtung zur Herstellung von Lipidvesikeln. Sie besteht im Prinzip aus einem ersten Vorratsbehälter für die polare und einem davon getrennten, zweiten Vorratsbehälter für die weitgehend apolare, lipidhältige Phase; einem Sammelbehälter zur Aufnahme der erzeugten Vesikelpräparation; einem Leitungssystem, welches vom ersten Vorratsbehälter (polare Phase) zum Sammelbehälter führt und welches an mindestens einer Stelle der bzw. jeder Leitung eine definierte, seitlich angeordnete Öffnung oder Bohrung zum Eintritt der lipidhältigen Phase aufweist; einem weiteren Leitungssystem, welches vom zweiten Vorratsbehälter (lipidhältige Phase) zu mindestens einer dieser seitlichen Öffnungen bzw. Bohrungen des ersten Leitungssystems führt; sowie Mittel zur Erzeugung der erforderlichen Flüssigkeitsströme, Strömungsprofile und Drücke für den kontrollierten Transport der Phasen und der entstehenden Phasendispersion.

Bevorzugt befinden sich jener Teil des Leitungssystems für die polare Phase, der die mindestens eine seitliche Öffnung aufweist sowie der damit verbundene Teil des Leitungssystems für die lipidhältige Phase in einem Kreuzstrommodul, welches eine technisch vorgefertigte Einheit sein kann, die sich einfach und rasch als verbindendes Kernstück in das Leitungssystem der beiden Phasen integrieren lässt. Es ist bevorzugt, dass zumindest innerhalb dieses Kreuzstrommoduls die Zufuhrleitungen für die polare und die lipidhältige Phase aus stabilem und chemisch widerstandsfähigem Material, beispielsweise aus Edelstahl oder Hartkunststoff, bestehen und dicht und unverrutschbar, beispielsweise durch Schweissen, miteinander verbunden sind. Für diskontinuierlichen oder semikontinuierlichen Betrieb kann der erste Vorratsbehälter ausserdem gleichzeitig als Sammelbehälter fungieren, wobei zumindest ein Teil des vom Vorratsbehälter wegführenden Leitungssystem im Kreis geführt wird und wieder in den Vorratsbehälter mündet, sodass wenigstens ein Teil der polaren Phase zirkuliert und inklusive neu gebildeter Lipidvesikel in den Vorratsbehälter/Sammelbehälter zurückgeführt wird.

### KURZE BESCHREIBUNG DER ABBILDUNGEN

- Fig. 1A: zeigt eine schematische Darstellung (in Draufsicht) eines Kreuzstrommoduls mit einer Flüssigkeitsleitung für die polare Phase (vertikal dargestellt) und einer seitlichen Zuleitung für die lipidhältige Phase (horizontal dargestellt); Fliessrichtungen werden durch Pfeile angedeutet;
- Fig. 1B: zeigt eine Querschnittsansicht des Kreuzstrommoduls der Fig. 1A;
- Fig. 1C: zeigt eine Querschnittsansicht eines Kreuzstrommoduls mit T-förmiger Anordnung der Zufuhrleitungen für die polare und lipidhältige Phase;
- Fig. 2: zeigt eine schematische Darstellung der erfindungsgemässen Vorrichtung für einen (z.B. kontinuierlichen) Betrieb ohne Rückführung der polaren Phase bzw. Phasendispersion;
- Fig. 3: zeigt eine schematische Darstellung der Vorrichtung aus Fig. 2 für einen (z.B. diskontinuierlichen) Betrieb mit Rückführung der polaren. Phase bzw. Phasendispersion in den Vorratsbehälter;
- Fig. 4: zeigt Grössenverteilungen von Liposomenpräparationen hergestellt nach dem Direktverfahren ohne Rücklauf (Kurve B) und nach einem zirkulierenden Verfahren (Kurve A) mit Rückführung der Phasendispersion in den Ausgangsbehälter und anschliessender weiterer Beladung mit Lipidphase (Ordinate: Prozent der Lipidvesikelpopulation; Abszisse: Durchmesser der Lipidvesikel in nm);
- Fig. 5 und Fig. 5A: zeigen Grössenverteilungen in Liposomenpräparationen (20 µmol DPPC/ml polare Phase) hergestellt bei verschiedenen Dosierdrücken (1.2 bar und 2.4 bar bzw. 2.5 und 4.5 bar) der lipidhältigen Phase (Ordinate: Prozent der Lipidvesikelpopulation; Abszisse: Durchmesser der Lipidvesikel in nm);
- Fig. 6: zeigt Grössenverteilungen von Liposomenpräparationen (10 µmol DPPC/ml polare Phase), hergestellt bei verschiedenen Dosierdrücken (0.3, 1.2 und 2 bar) der lipidhältigen Phase (Ordinate: Prozent der Lipidvesikelpopulation; Abszisse: Durchmesser der Lipidvesikel in nm);
- Fig. 7: zeigt die Grössenverteilungen innerhalb von Liposomenpräparationen in Abhängigkeit vom Verhältnis der eingesetzten Lipidmenge (in µmol) pro eingesetztem Volumensteil (in ml) der polaren Phase (5, 10 und 20 µmol/ml) bei ansonsten konstanten Bedingungen (Ordinate: Prozent der Lipidvesikelpopulation; Abszisse: Durchmesser der Lipidvesikel in nm);
- Fig. 8: zeigt die Reproduzierbarkeit der Vesikelgrössenverteilung unter gleichbleibenden Bedingungen für drei Versuchsreihen (Ordinate: Prozent der Lipidvesikelpopulation; Abszisse: Durchmesser der Lipidvesikel in nm);
- Fig. 9: zeigt einen Vergleich der Grössenverteilung der Lipidvesikel zwischen einem einstufigen Direktverfahren (Kurve A) und einem Verfahren (Kurve B) mit erhöhtem Mischungsverhältnis (Dosiervolumen der Lipidphase pro Volumenseinheit polarer Phase) und anschliessender Verdünnung der Phasendispersion (Ordinate: Prozent der Lipidvesikelpopulation; Abszisse: Durchmesser der Lipidvesikel in nm);
- Fig. 9A: zeigt einen Vergleich der Grössenverteilung der Lipidvesikel zwischen drei Verfahren mit erhöhtem Mischungsverhältnis (Dosiervolumen der Lipidphase pro Volumenseinheit polarer Phase) und anschliessender Verdünnung der Phasendispersion (Ordinate: Prozent der Lipidvesikelpopulation; Abszisse: Durchmesser der Lipidvesikel in nm);
- Fig. 10: zeigt die Grössenverteilung der Lipidvesikel in zwei Liposomenpräparationen, hergestellt mit Kreuzstrommodulen mit Bohrungen von 150 und 250 µm Durchmesser (Ordinate: Prozent der Lipidvesikelpopulation; Abszisse: Durchmesser der Lipidvesikel in nm);
- Fig. 11: zeigt die Grössenverteilung der Lipidvesikel in zwei Liposomenpräparationen, hergestellt mit Kreuzstrommodulen (250 µm Bohrung) mit einem Produktionsvolumen von 0.3 L (Kurve A) und einem Produktionsvolumen von 2.5 L (Kurve B). (Ordinate: Prozent der Lipidvesikelpopulation; Abszisse: Durchmesser der Lipidvesikel in nm);
- Fig. 12: zeigt Fluss-/Druck-Kurven von 92 Vol.% Ethanol bei 55°C bei Verwendung eines Kreuzstrommoduls mit Bohrungen von 150, 200 und 250 µm Durchmesser;
- Fig. 13: zeigt Flussraten von PBS als polarer Phase bei unterschiedlichen Leitungsdurchmessern (6, 8 und 10 mm; Pumpe: Schlauchquetschpumpe Type lsmatec SA0702).

### DETAILLIERTE BESCHEIBUNG DER ERFINDUNG

Als Trägermedium der polaren Phase werden sowohl für kosmetische als auch für pharmazeutische Präparationen, insbesondere solche für medizinische Anwendungen, bevorzugt wässrige Systeme, beispielsweise reines Wasser, PBS (Phosphat-gepufferte, physiologische Kochsalzlösung), physiologische Kochsalzlösung oder ein anderer geeigneter und pharmazeutisch zulässiger Puffer, gegebenenfalls mit üblichen Zusätzen wie Konservierungsstoffen, Duftstoffen, Farbstoffen, und dergleichen eingesetzt.

Das Trägermedium der lipidhältigen Phase wird bevorzugt aus solchen atoxischen, pharmazeutisch zulässigen, organischen Lösungsmitteln oder Lösungsmittelgemischen ausgewählt, die sich güt zum Lösen der für den jewiligen Einsatzzweck gewählten Lipide oder Lipidmischungen und der gegebenenfalls zusätzlichen Substanzen eignen. Solche zusätzlichen Substanzen können z.B. virale, fusogene Peptide wie etwa Influenza-Hämagglutinin, oder zell-spezifische Marker wie z.B. Antikörperfragmente, oder lipophile Wirkstoffe wie z.B. Econazol und dergleichen sein. Als bevorzugte Lösungsmittel kommen niedrige Alkohole (1 - 6 Kohlenstoffatome), wie Methanol, Ethanol, n-Propanol, Isopropanol, n-Butanol in Frage, wobei diese Lösungsmittel einzeln, im Gemisch und/oder gegebenenfalls zusammen mit einem geeigneten Puffer eingesetzt werden können.

Die Lipidvesikel sind nicht auf bestimmte Lipide oder Lipidzusammensetzungen beschränkt. Sie können je nach beabsichtigter Verwendung einfache und/oder komplexe Lipide, insbesondere Phospholipide, Glycolipide, derivatisierte Lipide, andere natürliche oder synthetische Lipide kationischer, anionischer und/oder ladungsneutraler Natur enthalten. Solche Lipide sind im Stand der Technik bekannt. Es können auch Lipoproteine oder Lipopolysaccharide mit der erfindungsgemässen Herstellungsmethode in die Membran der Lipidvesikel eingebaut werden. Membranstabilisierende Agenzien wie Cholesterol oder Cholesterolderivate bzw. Polyethylenglykol und dessen Derivate können zugesetzt werden.

Der Begriff "Lipidvesikel", wie er im Rahmen der vorliegenden Erfindung gemeint ist, umfasst sowohl reine, ausschliesslich aus Lipiden gebildete, Lipidvesikel, auch als Liposomen bezeichnet, als auch Lipidvesikel, die mit zell-spezifischen Markern, beispielsweise Zytokinen, Wachstumshormonen, Antikörpern oder Antikörperfragmenten, bestückt sind und häufig als Immunoliposomen bezeichnet werden, oder Lipidvesikel, die mit viralen Proteinen bzw. Antigenen, beispielsweise Hämagglutinin, bestückt sind und üblicherweise als Virosomen bezeichnet werden. Darüber hinaus können Lipidvesikel sowohl virale Antigene als auch spezifische Marker in der Vesikelmembran enthalten.

Unter "nativer Lipidvesikelpräparation" ist hierin eine Lipidvesikelsuspension zu verstehen, die unter scherkraftarmen oder scherkraftfreien Bedingungen unmittelbar und ohne Einwirkung zusätzlicher Hilfsmittel wie z.B. Ultraschall oder mechanischer Rühr- oder Dispergierhilfen nur durch das erfindungsgemässe Aufeinandertreffen der polaren und lipidhältigen Phasen entstanden ist und - mit Ausnahme einer allfälligen nachfolgenden Verdünnung mit einem polaren Lösungsmittel - keiner weiteren Nachbehandlung, insbesondere keiner Nachbehandlung zur Einstellung einer gewünschten Vesikelgrösse oder Vesikelgrössenverteilung, unterzogen worden ist.

Mit "gewünschter Substanz" ist eine beliebige chemische Substanz oder Verbindung gemeint, mit der sich Lipidvesikel beladen lassen, wobei die Substanz entweder aussen an der Vesikelmembran haftet und/oder in die Vesikelmembran integriert und/oder im Innenraum der Lipidvesikel eingeschlossen ist, und mit Hilfe der Lipidvesikel an einen gewünschten Zielort in vitro und/oder in vivo gebracht werden kann. Diese Substanz kann irgendeine Testsubstanz für beispielsweise wissenschaftliche Zwecke, oder eine pharmazeutisch aktive Substanz, ein medikamentöser Wirkstoff, ein Placebo, eine Substanz für kosmetische Zwecke, ein Farbstoff, eine radioaktivmarkierte oder fluoreszenz-markierte Verbindung für therapeutische oder diagnostische Anwendungen, oder eine andere chemische Verbindung oder Mischung von chemischen Verbindungen sein.

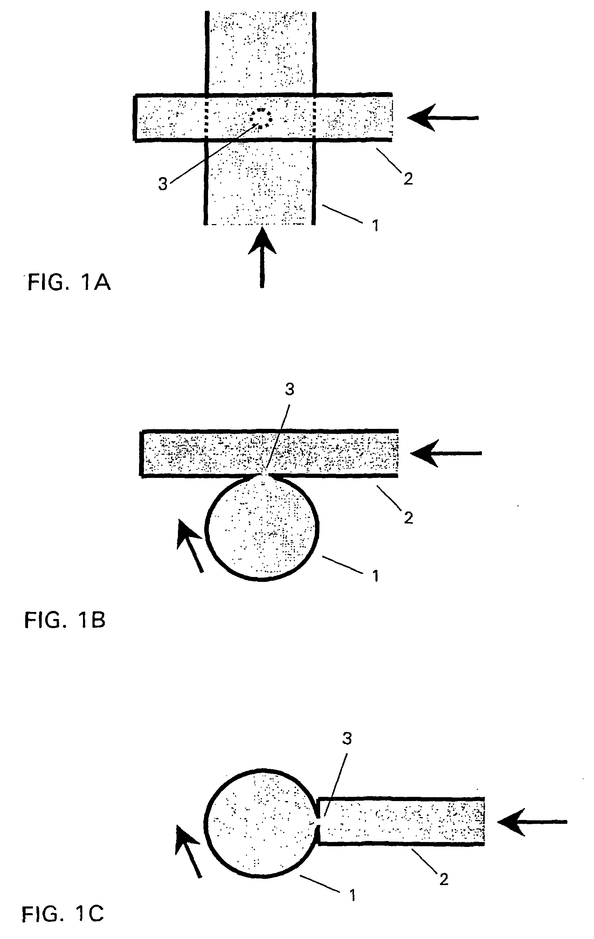

Das der Erfindung zugrunde liegende Prinzip des Aufeinandertreffens der polaren (z.B. wässrigen) Phase und der Lipidphase ist schematisch in Fig. 1A, Fig. 1B und Fig. 1C dargestellt. Eine im Inneren hohle Flüssigkeitsleitung 2, welche die organische Lipidphase in Richtung des horizontalen Pfeils transportiert, ist mit der Aussenseite einer ebenfalls im Inneren hohlen Flüssigkeitsleitung 1 verbunden, welche die polare (wässrige) Phase transportiert. Im Bereich der Kontaktstelle zwischen den beiden Leitungen befindet sich mindestens eine Bohrung oder Öffnung 3, welche durch die Seitenwand der Leitung 1 und durch die angrenzende Seiten- oder Stirnwand der Leitung 2 hindurchreicht und eine flüssigkeitsdurchlässige Verbindung zwischen dem Inneren der Leitung 1 und dem Inneren der Leitung 2 herstellt. Dabei kann die Leitung 2 die Leitung 1 überkreuzen (Kreuzstrommodul), wie in Fig. 1A und 1B schematisch dargestellt, oder mit ihrer Stirnseite direkt an der Seitenwand der Leitung 1 dicht und unverrutschbar befestigt, z.B. angeschweisst, sein, wie in Fig. 1C schematisch dargestellt.

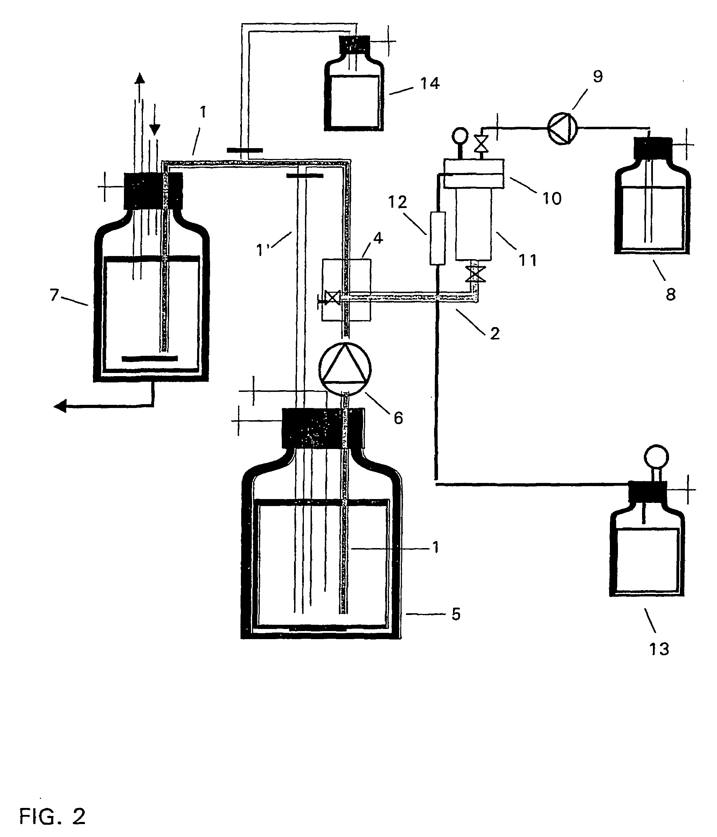

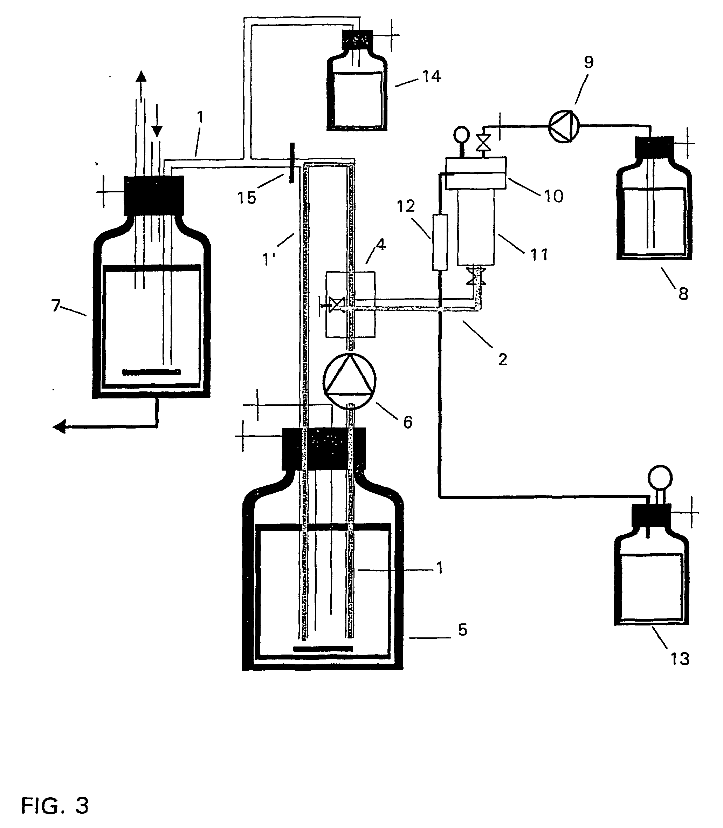

Kernstück der vorliegenden Erfindung ist eine Vorrichtung zur Herstellung der Lipidvesikel, wie sie beispielhaft in Fig. 2 und Fig. 3 dargestellt ist. In einem Kreuzstrommodul 4 ist eine Flüssigkeitsleitung 2 (Lipidphase) mit einer Leitung 1 (polare Phase) so verbunden, dass sie mit ihren Aussenseiten eine gemeinsame Kontaktfläche bilden und gegebenenfalls einander überlagern, wobei es unerheblich ist, in welchem Winkel sich die beiden Leitungen überlagern oder überkreuzen. Prinzipiell können sie auch parallel oder aber T-förmig zueinander angeordnet und miteinander verbunden sein, wie beispielsweise in Fig. 1C dargestellt. Wesentlich ist jedoch, dass die beiden Leitungen im Bereich ihrer gemeinsamen Kontaktfläche mindestens eine gemeinsame Öffnung 3, beispielsweise eine Bohrung aufweisen, welche die Innenräume der beiden Leitungen 1 und 2 miteinander verbindet und den Durchtritt von Flüssigkeit erlaubt. Durch die Öffnung 3 wird die durch die Leitung 2 fliessende Lipidphase unter mässigem Überdruck von vorzugsweise 0.1 bis 15 bar in die an der Öffnung 3 vorbeiströmende polare Phase eingespeist. Dabei ist die gemeinsame Öffnung 3 so angeordnet ist, dass die organische Flüssigkeitsphase aus der Leitung 2 quer, vorzugsweise im wesentlichen senkrecht, zur Fliessrichtung der in Leitung 1 fliessenden polaren Flüssigkeitsphase durch die Öffnung 3 hindurchtreten und, bevorzugt in Form eines Sprühnebels, in die an der Öffnung 3 vorbeiströmende polare Flüssigkeitsphase eindringen kann.

"Im wesentlichen senkrecht" bedeutet dabei, dass das Einspeisen bzw. Einsprühen der Lipidphase nicht in oder gegen die Strömungsrichtung der polaren Phase erfolgt, sondern quer dazu in einem, vorzugsweise rechten, Winkel, sodass die Lipidphase den Strom der polaren Flüssigkeitsphase von der Seite, also vom Aussenrand, her in Richtung Mitte hin penetriert. Es bedeutet ausserdem, dass im Falle des Vorliegens einer Sprühnebelcharakteristik, wie in den Abbildungen 1A bis 1C schematisch dargestellt, nur ein Teil der eingesprühten Lipidphase tatsächlich in einem rechten Winkel zur Strömungsrichtung der polaren Phase in diese eindringt, während der überwiegende Teil mehr oder weniger stark davon abweicht. Im Bereich der gemeinsamen Öffnung 3 sind die Leitungen 1 und 2 frei von zusätzlichen Einrichtungen, insbesondere frei von Rühr- oder Dispergierhilfen.

Fig. 2 zeigt beispielhaft eine Anordnung der erfindungsgemässen Vorrichtung zur Durchführung eines kontinuierlichen oder semikontinuierlichen Herstellungsverfahrens für Lipidvesikel, ohne Rückführung der entstehenden Vesikelsuspension in den Ausgangsbehälter. Da diese Art der Herstellung im Wesentlichen aus einem einzigen Verfahrensschritt besteht, nämlich aus der Zusammenführung der Lipidphase und der polaren Phase, wird dieses Verfahren im Nachfolgenden auch als "einstufiges Verfahren bezeichnet.

Die polare Phase wird aus einem ersten Vorratsbehälter 5 mittels einer Pumpe 6, die vorzugsweise möglichst pulsationsarm arbeitet und möglichst wenig Kavitation und Scherkräfte erzeugt, durch die Flüssigkeitsleitung 1 in Richtung Kreuzstrommodul 4 und weiter in den Sammelbehälter 7 gefördert, wobei die Pumpe 6 zwischen dem Vorratsbehälter 5 und dem Kreuzstrommodul 4 angeordnet ist. Im Betrieb ist die Pumpe 6 vorzugsweise so eingestellt, dass in der Leitung 1 zwischen dem Vorratsbehälter 5 und dem Sammelbehälter 7 wenigstens im Nahbereich vor und nach der Öffnung 3 eine laminare oder annähernd laminare Strömung vorliegt. Es ist vorzugsweise ausserdem darauf zu achten, dass keine Luftblasen in der Leitung 1 eingeschlossen werden, die die Ausbeute an intakten Vesikeln und damit den Einschlussgrad an Wirkstoff aus der polaren Phase schmälern könnten. Beim Durchströmen des Kreuzstrommoduls 4 wird die polare Phase mit der organischen, lipidhältigen Phase beladen, wobei die lipidhältige Phase unter Druck durch die Öffnung 3 im wesentlichen senkrecht zur Fliessrichtung der polaren Phase eingespeist wird. Es zeigt sich, dass die Lipide bereits innerhalb des Kreuzstrommoduls 4 präzipitieren und sich zu Vesikeln schliessen, wobei sie einen Teil der polaren Phase samt gegebenenfalls darin gelösten Substanzen einschliessen.

Vorzugsweise sind die Volumina und Strömungsgeschwindigkeiten der lipidhältigen und der polaren Phase so aufeinander abzustimmen, dass ungenutzte Überschüsse einer der beiden Phasen weitestgehend vermieden werden. Die lipidhältige Phase wird aus einem zweiten Vorratsbehälter 8 mit einer Pumpe 9 über einen Filter 10 und ein Rückschlag- oder sonstiges drucksperrendes Ventil in einen Zwischenbehälter 11 gepumpt. Der Zwischenbehälter 11 ist in diesem Beispiel über einen zwischengeschalteten Filter 12 mit einer Druckquelle 13 verbunden, beispielsweise einem Druckbehälter, der bevorzugt ein Inertgas, beispielsweise Stickstoffgas, unter Druck enthält und mit dessen Hilfe die lipidhältige Phase vom Zwischenbehälter 11 über ein regelbares Ventil in Richtung Kreuzstrommodul 4 gepresst wird.

Falls aufgrund der Lipidzusammensetzung und/oder der Wahl der liposomal einzuschliessenden Substanz kein Oxidationsschutz nötig ist, kann auch Pressluft eingesetzt werden. Alternativ dazu kann die lipidhältige Phase mittels einer Pumpe vom Zwischenbehälter 11 über die Leitung 2 zum Kreuzstrommodul 4 befördert werden. Sobald die polare Phase die Öffnung 3 im Kreuzstrommodul 4 passiert, wird ein regelbares Ventil in der Leitung 2 geöffnet und die lipidhältige Phase durch die Öffnung 3 in die Leitung 1 und damit in die polare Phase gepresst bzw. gepumpt. Zur Prozess- und Qualitätskontrolle kann zudem stromabwärts vom Kreuzstrommodul 4 eine Probeentnahmevorrichtung 14 vorgesehen sein.

In Fig. 3 wird die Anordnung aus Fig. 2 mit einer Rückführung der im Kreuzstrommodul 4 entstehenden Phasendispersion in den ersten Vorratsbehälter 5 (zirkulierendes Verfahren) dargestellt. Stromabwärts vom Kreuzstrommodul 4 wird zumindest ein Teil der Phasendispersion durch eine Sperre 15, beispielsweise ein Ventil, einen Schieber, eine Klemme oder dergleichen, vor dem Sammelbehälter 7 abgezweigt, über eine Rücklaufleitung 1' in den ersten Vorratsbehälter 5 rückgeführt und mittels Pumpe 6 wieder dem Kreuzstrommodul 4 zugeleitet, wo sie erneut mit lipidhältiger Phase beladen wird. Sobald die gewünschte Endkonzentration an Lipid oder Lipidvesikeln in der polaren Phase erreicht und/oder die lipidhältige Phase aufgebraucht ist, wird die Zirkulation gestoppt und die entstandene Phasendispersion entweder in den Sammelbehälter 7 übergeführt oder im Vorratsbehälter 5 gesammelt. Die erfindungsgemässe Vorrichtung, wie sie beispielhaft in den Figuren 2 und 3 dargestellt ist, kann nach bekannten Methoden der Steriltechnik keimfrei und pyrogenfrei betrieben werden. Sie kann beispielsweise thermisch oder chemisch sterilisiert werden und die Ausgangsmaterialien (polare Phase, lipidhältige Phase, gegebenenfalls Pressluft oder Inertgas) können über geeignete Sterilfiltersysteme den jeweiligen Vorratsbehältern 5, 8, dem Kreuzstrommodul 4 und dem anschliessenden Sammelbehälter 7 zugeführt werden. Die auf diese Weise hergestellten, nativen Lipidvesikel-Präparationen brauchen dann keinem weiteren Entkeimungsschritt mehr unterzogen zu werden.

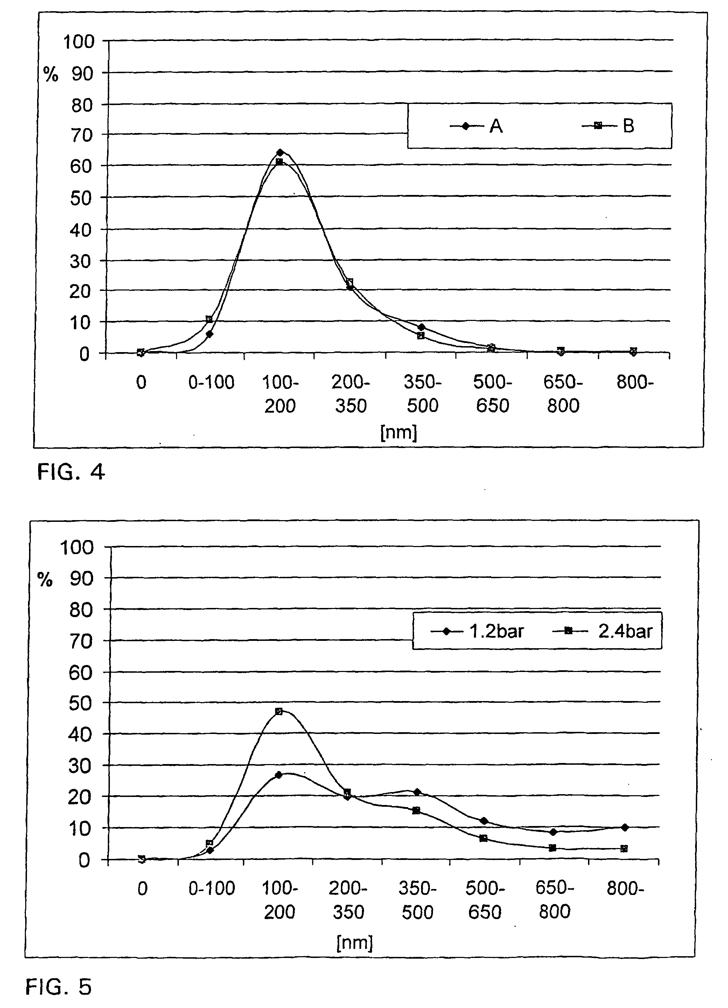

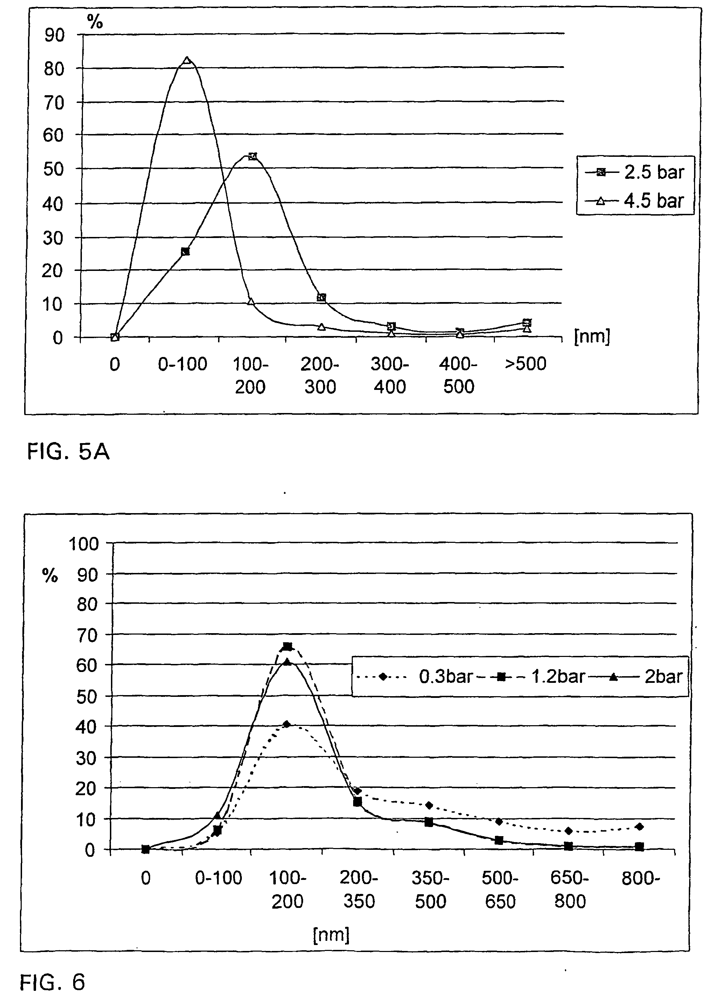

Die Grössenverteilung innerhalb der Vesikelpräparationen wird beim erfindungsgemässen Verfahren von zwei Faktoren massgeblich beeinflusst: einerseits von der je Volumensteil der polaren Phase zudosierten Menge an Lipid und/oder Lösungsmittel und andererseits vom Dosierdruck. Aus Fig. 5 und Fig. 6 ist zu ersehen, dass durch Erhöhung des Dosierdrucks der mittlere Durchmesser der Lipidvesikel abnimmt und die Grössenverteilung innerhalb der Vesikelpopulation weniger streut und somit homogener ist. Unter den Verfahrensbedingungen der Fig. 6 haben beispielsweise zwei Drittel aller erzeugten Liposomen einen Durchmesser von 100 bis 200 nm. Der Unterschied zwischen den Verfahrensbedingungen der Fig. 5 und der Fig. 6 besteht vor allem in der eingesetzten Lipidmenge je Volumenseinheit der polaren Phase. In Fig. 5 und Fig. 5A wurden 20 µmol Lipid pro ml polarer Phase eingesetzt, in Fig. 6 hingegen nur 10 µmol/ml. Es hat sich gezeigt, dass es bei geringeren Dosierdrücken von Vorteil ist, bei der gewählten Lipidzusammensetzung (Ergebnisse Fig. 4 bis 11) nicht mehr als 10 µmol Lipid pro ml polarer Phase zuzudosieren, um homogene Vesikelpräparationen mit geringer Streuung in der Grössenverteilung zu erhalten. Durch Erhöhung des Dosierdrucks lässt sich aber nicht nur die Verfahrenseffizienz steigern, sondern auch bei gesteigerter Lipidmenge (je Volumenseinheit der polaren Phase) eine geringe Streubreite der erzeugten Vesikel erreichen (Fig. 5A).

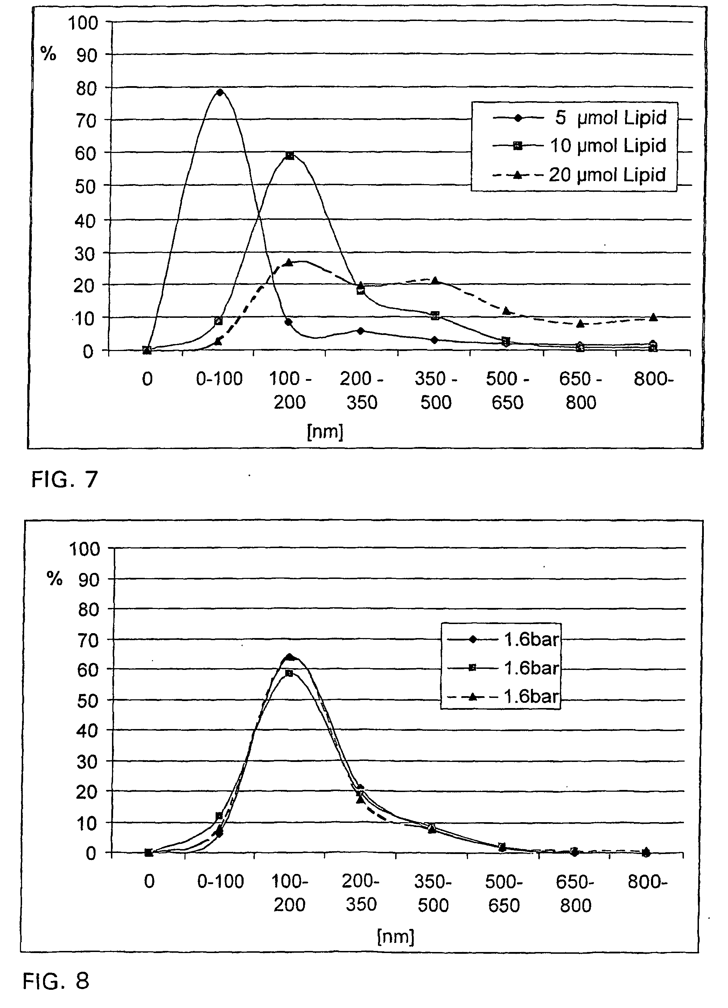

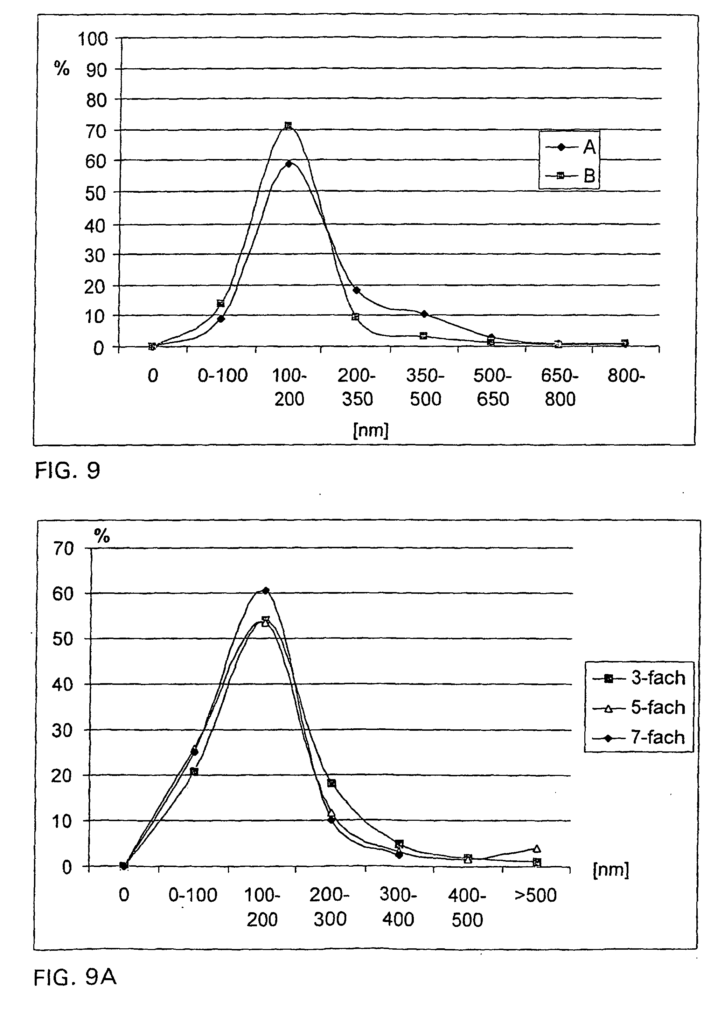

Aus Fig. 7 ist ersichtlich, dass mit steigendem Lipidanteil je Volumenseinheit polarer Phase, ausgedrückt in µmol eingesetzten Lipids pro ml eingesetzter polarer Phase, bei konstanten Bedingungen der übrigen Prozessparameter die durchschnittliche Grösse der Lipidvesikel und die Streuung der Grössenverteilung zunimmt. Diesem Effekt kann man jedoch durch Erhöhung des Dosierdrucks der lipidhältigen Phase entgegenwirken, sodass sich, wie in Fig. 9 dargestellt, trotz beträchtlicher Erhöhung des Lipidanteils dennoch eine hervorragende Homogenität und Grössenverteilung der Lipidvesikel ergibt. Eine Erklärungsmöglichkeit für dieses Phänomen könnte darin bestehen, dass bei höheren Dosierdrücken der auf die polare Phase einwirkende "Sprühnebel" der lipidhältigen Phase noch feiner ist und daher eine grössere Oberfläche aufweist. Ausserdem dürfte auch die Eindringtiefe des "Sprühnebels" in die polare Phase erhöht sein, sodass sich insgesamt daraus das beobachtete, reproduzierbare Phänomen ableiten lässt. Vergleichsversuche haben jedenfalls ergeben, dass durch Variation der Fliessgeschwindigkeit der polaren Phase der zuvor erwähnte Lipideffekt nicht aufgehoben werden konnte. Die besten Werte hinsichtlich Homogenität der Vesikel wurden, zumindest bei Verwendung von DPPC als alleiniger Bilayer-bildender Lipidkomponente, bei Lipidkonzentrationen von 10 µmol/ml und darunter erzielt.

Weiters ist es bevorzugt, das zudosierte Volumen der organischen Phase im Falle von Ethanol als Lösungsmittel so zu wählen, dass die kalkulierte Endkonzentration an Ethanol (bei Verwendung von Ethanol mit einer Reinheit ≥ 90 Vol%) in der stromabwärts vom Kreuzstrommodul 4 vorliegenden Vesikeldispersion 10 Vol.%, vorzugsweise 7.5 Vol. % nicht übersteigt. Eine Überschreitung dieser Endkonzentration kann die Stabilität, Homogenität und Grösse der gebildeten Lipidvesikel im Sammelbehälter 7 ungünstig beeinflussen. Bei Makromolekülen, wie beispielsweise Proteinen, sind unter diesen Bedingungen des einstufigen Verfahrens (ohne Rückführung) Einschlussgrade von 10 bis 15 Gew.% der Gesamtmenge des zugesetzten, in der wässrigen Phase gelösten Makromoleküls erreichbar.

Eine erhebliche Ausbeutesteigerung lässt sich aber mittels einer bevorzugten Ausführungsform dieses Verfahrens erreichen. Dabei wird das Mischungsverhältnis an zudosierter lipidhältiger Phase zu vorbeiströmender polarer Phase auf einen Wert eingestellt, der die oben erwähnten bevorzugten Grenzwerte von 7.5 Vol.% Ethanol und 10 µmol Lipid je 1 ml der polaren Phase in der Vesikeldispersion weit, beispielsweise um das Zwei- bis Zehnfache, überschreitet. Anschliessend kann die hochkonzentrierte Vesikelsuspension mit einem polaren Lösungsmittel, bevorzugt dem Trägermedium der polaren Phase, bis zu der bevorzugten Endkonzentration von 7.5 - 10 Vol. % Ethanol verdünnt werden, um - falls erforderlich - die Stabilität und Homogenität der Präparation auch für einen längeren Zeitraum (beispielsweise für Lagerungszwecke) sicher zu stellen. Die Verdünnung kann bereits in der Leitung 1 stromabwärts vom Kreuzstrommodul, oder erst im Sammelbehälter 7 erfolgen.

Es hat sich gezeigt, dass auf diese Weise der Einschlussgrad insbesondere für Proteine um ein Vielfaches gesteigert werden konnte. So konnte durch Erhöhung des Dosiervolumens der Lipidphase um das Dreifache eine direkt proportionale Steigerung des Einschlussgrades um ebenfalls ca. das Dreifache, beispielsweise von 10-15 Gew.% rekombinanter h-SOD auf 30-50 Gew. % rh-SOD, erzielt werden. Eine Erhöhung des Mischungverhältnisses auf das Fünf- bis Zehnfache der im nicht-modifizierten Verfahren bevorzugten Grenzwerte konnte eine weitere Ausbeutesteigerung bewirken, sodass ein Ausnutzungsgrad resultierte, der dem theoretisch möglichen annähernd entsprach. Wesentlich ist dabei aber, dass die erzeugten Lipidvesikel trotz des erhöhten Mischungsverhältnisses die charakteristische Grössenverteilung, wie sie beispielsweise in Fig. 9 erkennbar ist, beibehalten, was ursprünglich keineswegs zu erwarten oder vorherzusehen war.

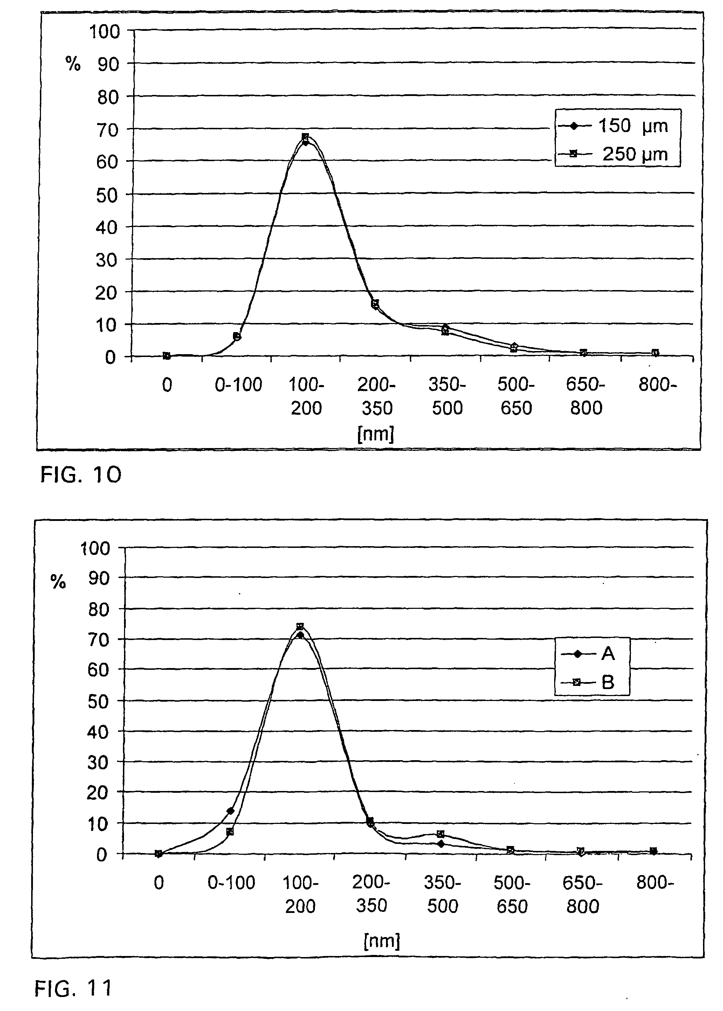

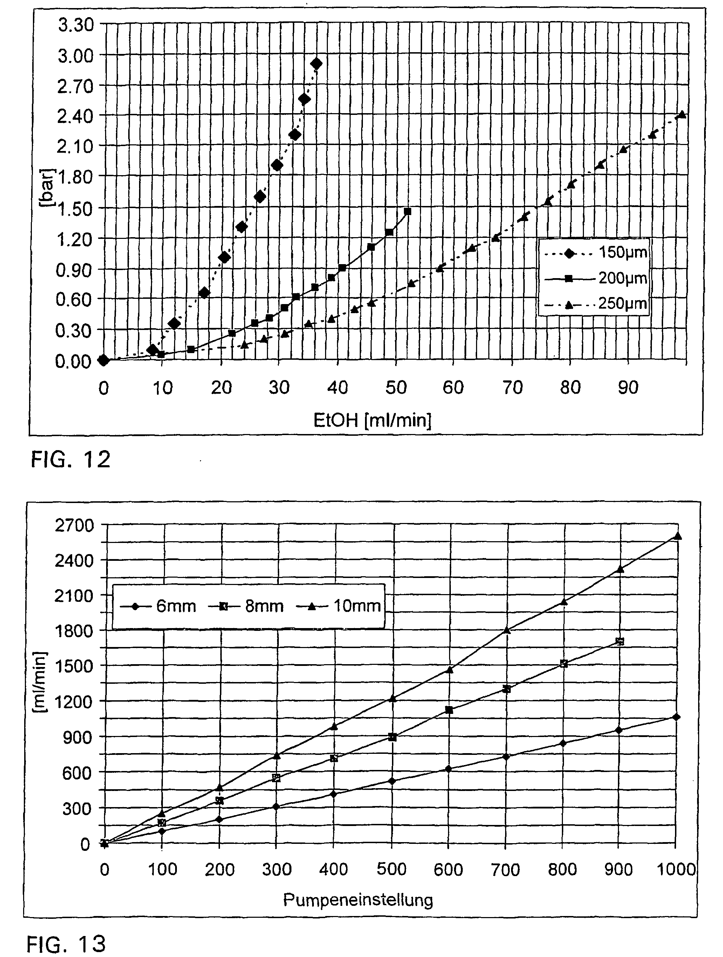

Die Erhöhung des Mischungsverhältnisses kann durch Erhöhung des Dosierdrucks der Lipidphase und gegebenenfalls zusätzlich durch Vergrösserung des Durchmessers der Öffnung 3 und/oder durch eine Erhöhung der Anzahl an Öffnungen 3 bewerkstelligt werden. Es ist auch möglich, den Zustrom der Lipidphase zu teilen und zwei oder mehr Leitungen 2 mit der Leitung 1 über Kontaktflächen mit Öffnungen 3 zu verbinden. Die Versuche haben gezeigt, dass eine Veränderung (Vergrösserung) des Lochdurchmessers der Öffnung 3 von beispielsweise 150 auf 250 µm zwar eine massive Steigerung des Durchsatzes an ethanolischer Phase ermöglicht (Fig. 12), jedoch offenbar keine wesentlichen Auswirkungen auf die durchschnittliche Grösse oder Grössenverteilung der Lipidvesikel mit sich bringt (Fig. 10). Es können daher je nach Anwendungszweck auch andere Durchmesser der Öffnung 3 gewählt werden. Als geeignet haben sich Durchmesser im Bereich von 50 - 1500 µm erwiesen. Es ist natürlich auch möglich und insbesondere für Produktionszwecke in grösserem Massstab vorteilhaft, den Flüssigkeitsstrom der polaren Phase zu teilen und zwei oder mehr Leitungen 1 vorzusehen, um so die Anzahl der Kontaktflächen und Öffnungen 3 noch weiter erhöhen zu können.
Zur gezielten Abstimmung der Volumensströme und Fliessgeschwindigkeiten der polaren und der lipidhältigen Phase aufeinander ist es von Vorteil, in vorausgehenden Experimenten die erreichbaren Flüsse in Abhängigkeit zur Pumpeneinstellung und den gewählten Leitungsquerschnitten zu messen und, wie beispielsweise in Fig. 13 für eine Vorrichtung im Labormassstab, graphisch darzustellen. Die Verfahrenstemperatur für die Vesikelherstellung ist naturgemäss von der chemischen Natur der eingesetzten Lipide und von der möglichen thermischen Empfindlichkeit der einzuschliessenden Substanz abhängig. Sie liegt aber stets über der Phasenübergangstemperatur der eingesetzten Lipide.

Mit dem erfindungsgmässen Verfahren sind native Vesikelpräparationen in einem einzigen Verfahrensschritt herstellbar, in denen zumindest 60 % aller Lipidvesikel (Figuren 4-11), gegebenenfalls mehr als 70 % (Fig. 6) aller Lipidvesikel, einen gewünschten, vorbestimmbaren Durchmesser aufweisen, der innerhalb einer Streubreite von maximal 250 nm, vorzugsweise von maximal 100 nm, liegt. Unter dem Begriff "Streubreite" ist in diesem Zusammenhang ein Grössenintervall der genannten Breite gemeint, innerhalb dessen die Durchmesser dieser zumindest 60 oder 70 % aller Vesikel verteilt liegen, also "streuen". Es lassen sich dementsprechend erfindungsgemässe Vesikelpräparationen herstellen, worin mindestens 60 % aller Vesikel einen Durchmesser im Bereich von beispielsweise 100 - 200 nm (Streubreite = 100 nm), oder mindestens 70 % einen Durchmesser im Bereich von 100 - 350 nm (Streubreite = 250 nm) aufweisen. Genausogut lassen sich aber auch Vesikelpräparationen herstellen, worin mindestens 60 % oder mindestens 70 % aller Vesikel einen Durchmesser im Bereich von 500- 600 nm (Streubreite 100 nm) oder 500 - 750 nm (Streubreite 250 nm) aufweisen. Das Verfahren lässt sich sowohl hinsichtlich der gewünschten Lipidzusammensetzung als auch hinsichtlich der optimalen Vesikelgrösse und der zu inkorporierenden Substanzen weitgehend an die verschiedensten Bedürfnisse kosmetischer, diagnostischer oder medizinisch-therapeutischer Anwendungen anpassen. Im Nachfolgenden wird die Erfindung anhand von Beispielen weiter erläutert.

### Beispiel 1: Inkorporation von rekombinanter humaner Superoxiddismutase (rh-SOD) in Lipidvesikel

Rekombinante humane Superoxiddismutase (rh-SOD) ist ein Protein mit einem Molekulargewicht von rund 32000 Dalton. Über die Vorteile einer liposomalen Verkapselung dieses Proteins und die dadurch ermöglichten medizinischen Anwendungen wird in WO96/14083 ausführlich berichtet. Da dieses Protein in der polaren Phase gelöst ist und nicht mit den Lipidmembranen interagiert, wird die rh-SOD "passiv" inkorporiert.

Einstufiges Verfahren (schematisch dargestellt in Fig. 2):
In 100 ml PBS (115 mg Na₂HPO₄, 20 mg KH₂PO₄; 800 mg NaCl; 20 mg KCI pH 7.2 - 7.4) als polarer Phase werden 1600 mg rh-SOD und in 7.5 ml Ethanol (Konzentration: 92 Vol.%) als organischer Phase werden 10 µmol/ml (bezogen auf das Volumen der polaren Phase) Bilayer-bildendes Lipid, beispielsweise 734 mg DPPC (Dipalmitoyl-phosphatidylcholin), zusammen mit 2.86 µmol/ml (110 mg) Cholesterol und 1.43 µmol/ml (38.5 mg) Stearylamin, gelöst. Die Lipidzusammensetzung, in diesem Fall DPPC, Chotesterol und Stearylamin im Verhältnis von 7:2:1 µmol/ml, kann jedoch sowohl hinsichtlich der Auswahl der Lipidkomponenten als auch hinsichtlich der Mengenverhältnisse der Lipidkomponenten zueinander variieren. Für viele Liposomenformulierungen sind jedenfalls Lipidgemische, wie in diesem Beispiel, zur stabilen Vesikelbildung besser geeignet als die Verwendung einer einzelnen Lipidkomponente allein. In den nachfolgenden Beispielen wurde stets eine Mischung aus Bilayer-bildendem Lipid (z.B. DPPC, DOPC, DMPC), Cholesterol und Stearylamin eingesetzt.

Die lipidhältige organische Phase (98 mg DPPC/ml Ethanol) wird in einem Kreuzstrommodul über eine Bohrung mit 250 µm Durchmesser mit einem Druck von 1.5 bar pumpenfrei mittels Drucküberlagerung aus der Stickstoff-Gasflasche in die vorbeiströmende polare Phase (16 mg rh-SOD/ml PBS) eingespeist und die entstandene Vesikeldispersion in den Sammelbehälter überführt. Als Transportleitungen für die polare und für die lipidhältige Phase werden Silikonschläuche verwendet. Der innendurchmesser der Schlauchleitung für die Förderung der polaren Phase vom Vorratsbehälter zum Kreuzstrommodul und vom Kreuzstrommodul zum Sammelbehälter beträgt 10 mm, derjenige für die Förderung der Lipidphase vom Zwischenbehälter bis zum Kreuzstrommodul beträgt 1.6 mm. Als Pumpe für die Beförderung der polaren Phase kommt eine Schlauchquetschpumpe vom Typ Ismatec SA 0702 zum Einsatz, als Pumpeneinstellung (siehe Fig. 13) wird 999 gewählt, entsprechend einer Pumpleistung von 2600 ml/min bei dem verwendeten Schlauchdurchmesser von 10 mm; die lipidhältige Phase wird vorzugsweise pumpenfrei durch Drucküberlagerung mittels Pressluft oder Inertgas, in diesem Beispiel mittels Stickstoffgas, zum Kreuzstrommodul befördert. Im Überstand der Präparation werden 14 bis 14.5 mg rh-SOD/ml gemessen. Demnach sind 150 bis 200 mg rh-SOD in die Liposomen eingeschlossen worden. Das entspricht einem Einschlussgrad von 9.5 -12.5 % der ursprünglich in PBS gelösten Menge an rh-SOD. Analoge Vergleichsversuche mit DOPC (Dioleoyl-phosphatidylcholin) und DMPC (Dimyristoyl-phosphatidylcholin) haben sehr ähnliche Ergebnisse erbracht (Resultate nicht dargestellt).

### Beispiel 2: Vergleich von einstufigem Verfahren (ohne Rücklauf) und Verfahren mit Rücklauf

Es wird gemäss Beispiel 1 vorgegangen, allfällige Abweichungen sind in der nachfolgenden Tabelle 1 angeführt. Die Versuchsanordnung für das Rezirkulationsverfahren entspricht der Vorrichtung, die schematisch in Fig. 3 dargestellt ist, jene für das einstufige Verfahren entspricht der Vorrichtung gemäss Fig. 2. Die Resultate sind in Tabelle 1 und Fig. 4 wiedergegeben.

**Tabelle 1:**

| Vergleich der Vesikelgrössenverteilung im einstufigen und rezirkulierenden Verfahren | | |
|---|---|---|
| Vesikelgrössenbereich [nm] | Prozent [%] aller Vesikel bei | |
| | VF* mit Rücklauf | VF* ohne Rücklauf ("einstufig") |
| 0 | 0 | 0 |
| 0-100 | 6 | 10.48 |
| 100-200 | 64.11 | 60.78 |
| 200-350 | 20.83 | 22.64 |
| 350-500 | 7.91 | 5.09 |
| 500-650 | 1.45 | 1.12 |
| 650-800 | 0.14 | 0.38 |
| 800- | 0.18 | 0.3 |

| **Lipidphase** | | |
|---|---|---|
| Lipid | 10 µmol DPPC / 1 ml polare Phase | |
| Bohrung | 250 µm | |
| Dosierung | Drucküberlagerung mit Stickstoff | |
| Dosierdruck | 1.6 bar | 11.5 bar |
| Fluss | 75 ml/min | |

| **Polare Phase** | | |
|---|---|---|
| Lösungsmittel | PBS | |
| Schlauch | Silikon; 6 mm Innendurchmesser | |
| Fluss | 1000 ml/min | 950 ml/min |

| | | |
|---|---|---|
| *VF = Verfahren | | |

Es zeigt sich, dass die Grössenverteilung in beiden Fällen nahezu identisch ist, wobei mehr als 60% aller gebildeten Vesikel einen Durchmesser von 100 bis 200 nm aufweisen.

### Beispiel 3: Einfluss des Dosierdrucks der lipidhältigen Phase auf Grösse und Grössenverteilung der Lipidvesikel

Beispiel 1 wird ohne rhSOD und mit folgenden Modifikationen wiederholt: Die polare Phase wird im Gegensatz zu Beispiel 1 im Kreis geführt (rezirkuliert). Die Lipidphase enthält insgesamt 1470 mg DPPC, entsprechend 20 µmol DPPC (Molekulargewicht von DPPC = 734) pro 1 ml polarer Phase. Dosierdrücke von 1.2 und 2.4 bar sowie 2.5 und 4.5 bar für die lipidhältige Phase werden miteinander verglichen.

**Tabelle 2:**

| Vergleich der Vesikelgrössenverteilung von Liposomenbatches (20 µmol Lipid/ml polare Phase) mit unterschiedlichen Dosierdrücken | | |
|---|---|---|
| Vesikelgrössenbereich [nm] | Prozent [%] aller Vesikel bei | |
| | 1.2 bar | 2.4 bar |
| 0 | 0 | 0 |
| 0-100 | 2.89 | 4.95 |
| 100-200 | 26.8 | 47 |
| 200-350 | 19.73 | 21.03 |
| 350-500 | 21.21 | 15.19 |
| 500-650 | 11.86 | 6.55 |
| 650-800 | 8.26 | 3.14 |
| 800- | 10.1 | 3.16 |

Die Vesikelgrössen wurden in diesem und allen anderen hierin beschriebenen Experimenten mittels Flow-Cytometrie, adaptiert nach Vorauer-Uhl et al. (Cytometrie 39(2): 166-71, 2000) ermittelt. Die Resultate sind in Tabelle 2 und 2a und in Fig. 5 und 5A wiedergegeben.

**Tabelle 2a:**

| Vergleich der Vesikelgrössenverteilung von Liposomenbatches (20 µmol Lipid/ml polare Phase) mit unterschiedlichen Dosierdrücken | | |
|---|---|---|
| Vesikelgrössenbereich [nm] | Prozent [%] aller Vesikel bei | |
| | 2.5 bar | 4.5 bar |
| 0 | 0 | 0 |
| 0-100 | 25.86 | 82.19 |
| 100-200 | 53.62 | 10.64 |
| 200-300 | 11.66 | 3.05 |
| 300-400 | 3.2 | 1.15 |
| 400-500 | 1.52 | 0.67 |
| 500- | 4.14 | 2.30 |

### Beispiel 4: Einfluss des Dosierdrucks der lipidhältigen Phase auf Grösse und Grössenverteilung der Lipidvesikel

Beispiel 1 wird ohne rhSOD und mit folgenden Modifikationen wiederholt: Die polare Phase wird im Gegensatz zu Beispiel 1 im Kreis geführt (rezirkuliert). Drei Versuchsansätze gleicher Lipidkonzentration werden miteinander verglichen, wobei Dosierdrücke von 0.3, 1.2 und 2.0 bar für die Einspeisung der lipidhältigen Phase in die polare Phase getestet werden. Die polare Phase enthält jeweils konstant 200 ml PBS, die Lipidphase enthält jeweils 1470 mg DPPC in 15 ml Ethanol (92 Vol. %), entsprechend 10 µmol DPPC (Molekulargewicht von DPPC = 734) pro 1 ml polarer Phase. Befördert wird die lipidhältige Phase in diesem Beispiel mit Hilfe einer Zahnradpumpe vom Typ Gather P 15133. Die Resultate sind in Tabelle 3 und in Fig. 6 wiedergegeben.

**Tabelle 3:**

| Grössenvergleich der Vesikel aus Liposomenbatches (10 µmol Lipid/ml polare Phase) hergestellt mit Dosierdrücken von 0.3, 1.2, 2.0 bar | | | |
|---|---|---|---|
| Vesikelgrössenbereich [nm] | Prozent [%] aller Vesikel bei | | |
| | 0.3 bar | 1.2 bar | 2.0 bar |
| 0 | 0 | 0 | 0 |
| 0-100 | 5.49 | 5.96 | 11.21 |
| 100-200 | 40.39 | 65.61 | 60.75 |
| 200-350 | 18.75 | 15.5 | 15.22 |
| 350-500 | 14.46 | 8.84 | 8.74 |
| 500-650 | 8.83 | 2.97 | 2.89 |
| 650-800 | 5.78 | 0.81 | 0.88 |
| 800- | 7.25 | 0.79 | 0.95 |
| | | | |
| Fluss (Lipidphase) | 11 ml/min | 23 ml/min | 31 ml/min |
| Fluss (polare Phase) | 2700 ml/min | 2700 ml/min | 2700 ml/min |
| Gesamtvolumen der polaren Phase | 200 ml | 200 ml | 200 ml |
| Versuchsdauer | 82 sec | 39 sec | 29 sec |

### Beispiel 5: Vergleich von Vesikelgrössen aus Versuchsansätzen mit unterschiedlicher Lipidkonzentration

Beispiel 1 wird ohne rhSOD und mit folgenden Modifikationen wiederholt: Die lipidhältige Phase enthält 734, 1470, bzw. 2940 mg DPPC, entsprechend den kalkulierten Konzentrationen von 5, 10 und 20 µmol DPPC pro 1 ml polare Phase. Die Resultate sind in Tabelle 4 und Fig. 7 wiedergegeben.

**Tabelle 4:**

| Vesikelgrössenvergleich von Liposomenbatches mit unterschiedlicher Lipidkonzentration | | | |
|---|---|---|---|
| Vesikelgrössenbereich in [nm] | Prozent [%] aller Vesikel bei | | |
| | 5 µmol Lipid /ml | 10 µmol Lipid /ml | 20 µmol Lipid /ml |
| 0 | 0 | 0 | 0 |
| 0-100 | 78.37 | 8.96 | 2.89 |
| 100-200 | 8.6 | 58.9 | 26.8 |
| 200-350 | 5.84 | 18.22 | 19.73 |
| 350-500 | 3.14 | 10.46 | 21.21 |
| 500-650 | 1.96 | 2.7 | 11.86 |
| 650-800 | 1.68 | 0.9 | 8.26 |
| 800- | 2.06 | 0.63 | 10.1 |

### Beispiel 6: Reproduzierbarkeit der Grössenverteilungen von Lipidvesikeln

In Tabelle 5 und Fig. 8 sind drei separate Vesikelpräparationen dargestellt, die unter identischen Bedingungen hergestellt wurden und in denen nahezu gleiche Grössenverteilungen der Lipidvesikel vorliegen. Die Vesikelpräparationen wurden gemäss Beispiel 1, jedoch ohne rhSOD hergestellt; allfällige Abweichungen davon sind in der nachfolgenden Tabelle 5 angeführt. Die polare Phase wird im Gegensatz zu Beispiel 1 im Kreis geführt (rezirkuliert).

**Tabelle 5:**

| Reproduzierbarkeit der Grössenverteilung von Lipidvesikeln (Dosierdruck 1.6 bar) | | | |
|---|---|---|---|
| Vesikelgrössenbereich | Prozent [%] aller Vesikel | | |
| | Präparat 1 | Präparat 2 | Präparat 3 |
| 0-100 | 6 | 12 | 8.17 |
| 100-200 | 64.11 | 58.33 | 63.92 |
| 200-350 | 20.83 | 19.3 | 17.24 |
| 350-500 | 7.91 | 8.43 | 7.77 |
| 500-650 | 1.45 | 1.93 | 1.91 |
| 650-800 | 0.14 | 0.38 | 0.7 |
| 800- | 0.18 | 0.13 | 0.83 |

| Lipidphase | | | |
|---|---|---|---|
| EtOH | 92 Vol. % | | |
| Lipid-dosierung | 10 µmol DPPC je 1 ml polare Phase | | |
| Dosierart | N₂-Gas | N₂-Gas | Pumpe* |
| Bohrung | 250 µm | | |
| Fluss | 75 ml/min | 75 ml/min | 75 ml/min |

| Polare Phase | | | |
|---|---|---|---|
| Tägermedium | PBS | | |
| Fluss | 2700 ml/min | | |
| Menge | 200 ml | 200 ml | 200 ml |
| Dauer | 12 sec | 12 sec | 12 sec |

| | | | |
|---|---|---|---|
| * Zahnradpumpe Modell Gather P15133 | | | |

### Beispiel 7: Modifiziertes einstufiges Verfahren mit erhöhtem Dosiervolumen der Lipidphase und nachfolgendem Verdünnen der entstandenen Vesikeldispersion

Beispiel 1 wird mit folgenden Modifikationen wiederholt:
Unter Verwendung einer Vorrichtung gemäss Fig. 2 werden im Sammelbehälter zwei Volumensteile (200 ml) der polaren Phase (PBS) und im Vorratsbehälter ein Volumensteil (100 ml) PBS vorgelegt. In den 100 ml PBS des Vorratsbehälters werden 1600 mg rh-SOD gelöst. Es wird nun - im Vergleich zu Beispiel 1 - die dreifache Lipidmenge (2205 mg Lipid) in der dreifachen Menge (22.5 ml) Ethanol (Konzentration: 92 Vol.%) gelöst. Die Strömungsgeschwindigkeit der polaren Phase wird so gesteuert, dass die gesamte Menge an Lipid/Ethanol-Lösung in den einen Volumensteil (100 ml) der polaren Phase eingebracht wird. Die im Kreuzstrommodul entstehende Phasendispersion wird in den Sammelbehälter weitergeleitet, wo der Überschuss an Ethanol sofort durch das vorgelegte polare Lösungsmittel PBS ausgeglichen wird. Durch dieses Verfahren werden 450 bis 600 mg rh-SOD in den Lipidvesikeln eingeschlossen. Das entspricht einer Inkorporationsrate von 28 bis 38 % bezogen auf die Gesamtmenge an vorgelegter rh-SOD.

Damit wird mit dieser Verfahrensvariante etwa die 3-fache Menge an Protein im Vergleich zum Verfahren nach Beispiel 1 eingeschlossen. Die mit diesem Verfahren erzielte Vesikelgrössenverteilung ist in der nachfolgenden Tabelle 6 sowie in Fig. 9 wiedergegeben. Wie aus Fig. 9 zu ersehen ist, übertrifft die mit dieser Präparationstechnik erhaltene Grössenverteilung der Lipidvesikel sogar jene aus dem nicht-modifizerten einstufigen Verfahren in Bezug auf die Homogenität und den Anteil an Vesikeln mit einem Durchmesser im Bereich von 100-200 nm.

**Tabelle 6:**

| Vergleich der Vesikelgrössenverteilung im einstufigen Verfahren bei normalem und erhöhtem Dosiervolumen der Lipidphase je Volumen der polaren Phase | | |
|---|---|---|
| Vesikelgrössenbereich | Prozent [%] aller Vesikel | |
| | normal | dreifach konzentriert |
| 0 | 0 | 0 |
| 0-100 | 8.96 | 14.02 |
| 100-200 | 58.9 | 71.31 |
| 200-350 | 18.22 | 9.5 |
| 350-500 | 10.46 | 3.28 |
| 500-650 | 2.7 | 1.22 |
| 650-800 | 0.9 | 0.52 |
| 800- | 0.63 | 0.75 |

| **Lipidphase** | | |
|---|---|---|
| Lipid | 10 µmol DPPC pro 1 ml polare Phase (bezogen auf die in Vorrats- und Sammelbehälter am Start insgesamt vorgelegte Menge an polarer Phase) | |
| Bohrung | 250 µm | |
| Dosierung | Drucküberlagerung mit Stickstoffgas | |
| Dosierdruck | 2.4 bar | |

| **Polare Phase** | | |
|---|---|---|
| Trägermedium | PBS | |
| Schlauch | Silikon; 6 mm Innendurchmesser | |
| Fluss | 400 ml/min | |

### Beispiel 7a: Modifiziertes einstufiges Verfahren mit erhöhtem Dosiervolumen der Lipidphase und nachfolgendem Verdünnen der entstandenen Vesikeldispersion

Beispiel 1 wird mit folgenden Modifikationen wiederholt:
Unter Verwendung einer Vorrichtung gemäss Fig. 2 werden im Sammelbehälter zwei, vier oder sechs Volumensteile (200 ml, 400 ml oder 600 ml) der polaren Phase (PBS) und im Vorratsbehälter ein Volumensteil (100 ml) PBS vorgelegt. In den 100 ml PBS des Vorratsbehälters werden 1600 mg rh-SOD gelöst. Es wird nun - im Vergleich zu Beispiel 1 - die drei-, fünf- oder siebenfache Lipidmenge (2205, 3670, bzw. 5138 mg Lipid) in der drei-, fünf- bzw. siebenfachen Menge (22.5, 37.5 bzw. 52.5 ml) Ethanol (Konzentration: 92 Vol.%) gelöst. Die Strömungsgeschwindigkeit der polaren Phase wird so gesteuert, dass die gesamte Menge an Lipid/Ethanol-Lösung in den einen Volumensteil (100 ml) der polaren Phase eingebracht wird. Die im Kreuzstrommodul entstehende Phasendispersion wird in den Sammelbehälter weitergeleitet, wo der Überschuss an Ethanol sofort durch das vorgelegte. polare Lösungsmittel PBS ausgeglichen wird. Durch dieses Verfahren werden 450 bis 600 mg (bei 3-facher Lipidmenge), 640 bis 720 mg (bei 5-facher Lipidmenge) bzw. 880 bis 960 mg (bei 7-facher Lipidmenge) rh-SOD in den Lipidvesikeln eingeschlossen. Das entspricht einer Inkorporationsrate von 28 bis 38 %, bezogen auf die Gesamtmenge an vorgelegter rh-SOD, bzw. 40 bis 45% bei der 5-fach und 55 bis 60% bei der 7-fach Dosierung.

Damit wird mit dieser Verfahrensvariante etwa die 3, 5, bzw. 7-fache Menge an Protein im Vergleich zum Verfahren nach Beispiel 1 eingeschlossen. Die mit diesem Verfahren erzielte Vesikelgrössenverteilung ist in der nachfolgenden Tabelle 6a sowie in Fig.9A wiedergegeben.

**Tabelle 6a:**

| Vergleich der Vesikelgrössenverteilung im einstufigen Verfahren bei normalem und erhöhtem Dosiervolumen der Lipidphase je Volumen der polaren Phase | | | |
|---|---|---|---|
| Vesikelgrössenbereich [nm] | Prozent aller Vesikel bei | | |
| | 3-fach | 5-fach | 7-fach |
| 0 | 0 | 0 | 0 |
| 0-100 | 20.8 | 25.86 | 25.26 |
| 100-200 | 53.93 | 53.62 | 60.57 |
| 200-300 | 18.09 | 11.66 | 9.9 |
| 300-400 | 4.99 | 3.20 | 2.35 |
| 400-500 | 1.98 | 1.52 | 1.27 |
| 500- | 1.15 | 4.14 | 1.48 |

| **Lipidphase** | | | |
|---|---|---|---|
| Lipid | 10 µmol DPPC pro 1 ml polare Phase (bezogen auf die in Vorrats- und Sammelbehälter am Start insgesamt vorgelegte Menge an polarer Phase) | | |
| Bohrung | 250 µm und 500µm | | |
| Dosierung | Drucküberlagerung mit Stickstoffgas | | |
| Dosierdruck | 2.5 bar | | |

| **Polare Phase** | | | |
|---|---|---|---|
| Trägermedium | PBS | | |
| Schlauch | Silikon; 6 mm Innendurchmesser | | |
| Fluss | 400 ml/min | | |

### Beispiel 8: Vergleich der Vesikelgrössenverteilung in Abhängigkeit zur Lochgrösse der Bohrung im Kreuzstrommodul

Beispiel 1 wird ohne rhSOD wiederholt; allfällige Abweichungen davon sind in der nachfolgenden Tabelle 7 angeführt. Die polare Phase wird im Gegensatz zu Beispiel 1 im Kreis geführt (rezirkuliert). Die Resultate sind Fig. 10 und in Tabelle 7 wiedergegeben.

**Tabelle 7:**

| Vesikelgrössenvergleich bei Verwendung von Kreuzstrommodulen mit unterschiedlichen Bohrungsdurchmessern | | |
|---|---|---|
| Vesikelgrössenbereich | Prozent [%] aller Vesikel bei einem Bohrungsdurchmesser von | |
| | 150 µm | 250mm |
| 0 | 0 | 0 |
| 0-100 | 5.96 | 6.3 |
| 100-200 | 65.61 | 67.01 |
| 200-350 | 15.5 | 16.34 |
| 350-500 | 8.84 | 7.47 |
| 500-650 | 2.97 | 2 |
| 650-800 | 0.81 | 0.67 |
| 800- | 0.79 | 0.74 |

| **Lipidphase** | | |
|---|---|---|
| Lipid | 10 µmol DPPC/ ml polare Phase | |
| Bohrung | 150 µm | 250 µm |
| Dosierung | Zahnradpumpe Typ Gather P15133 | |
| Dosierdruck | 1.2 bar | |
| Fluss | 23 ml/min | 67 ml/min |

| **Polare Phase** | | |
|---|---|---|
| Trägermedium | PBS | |
| Schlauch | Silikon; 10 mm Innendurchmesser | |
| Fluss | 2700 ml/min | |

Aus den Resultaten ist zu entnehmen, dass der Durchmesser der Dosieröffnung im Kreuzstrommodul, zumindest im Bereich der getesteten Durchmesser, keinen wesentlichen Einfluss auf die Grösse und die Grössenverteilung der erzeugten Lipidvesikel zu haben scheint. Weitere Versuche haben ergeben, dass insbesondere Bohrungen mit Durchmessern im Bereich von 50 bis 1500 µm für die Zwecke der vorliegenden Erfindung als geeignet erscheinen. Kleinere Bohrungsdurchmesser lassen sich mechanisch nur schwer herstellen und kommen daher weniger in Frage.

### Beispiel 9: Vergleich der Vesikelgrössenverteilung im 0.3 und 2.5 Liter Massstab

Beispiel 7 wird wiederholt, wobei in einem ersten Versuchsansatz 300 ml an polarer Phase (wie in Beispiel 7) und einem zweiten Versuchsansatz 2500 ml an polarer Phase unter ansonsten identischen Bedingungen eingesetzt werden. Die Lipid- und Ethanolkonzentrationen entsprechen jenen, die in Beispiel 7 angeführt sind, wobei im 2500 ml Versuchsansatz natürlich auch das Flüssigkeitsvolumen der ethanolischen Lipidphase um den selben Faktor höher ist (d.h. 187.5 ml).

**Tabelle 8:**

| Vesikelgrössenverteilung bei Massstabsvergrösserung | | |
|---|---|---|
| Vesikelgrössenbereich | Prozent (%) aller Vesikel | |
| | 0.3 Liter | 2.5 Liter |
| 0 | 0 | 0 |
| 0-100 | 14.02 | 7.18 |
| 100-200 | 71.31 | 73.51 |
| 200-350 | 9.5 | 10.16 |
| 350-500 | 3.28 | 6.14 |
| 500-650 | 1.22 | 1.24 |
| 650-800 | 0.52 | 0.84 |
| 800- | 0.75 | 0.93 |

| **Lipidphase** | | |
|---|---|---|
| Lipid | 10 µmol DPPC pro 1 ml polare Phase (bezogen auf die in Vorrats- und Sammelbehälter am Start insgesamt vorgelegte Menge an polarer Phase) | |
| Bohrung | 250 µm | |
| Dosierung | Drucküberlagerung mit Stickstoffgas | |
| Dosierdruck | 2.4 bar | |

| **Polare Phase** | | |
|---|---|---|
| Trägermedium | PBS | |
| Schlauch | Silikon; 6 mm Innendurchmesser | |
| Fluss | 400 ml/min | |

### Beispiel 10: Ermittlung von Druck/Fluss-Kurven für die Flüssigkeitsphasen

Um die Flüssigkeitsströme der polaren und der ethanolischen Lipidphase aufeinander abstimmen zu können, wurden mit den verwendeten Pumpen entsprechende Fluss- bzw. Druck/Fluss-Kurven ermittelt und in Form von Diagrammen dargestellt. Zum Einsatz kamen einerseits eine Schlauchquetschpumpe Modell Ismatec SA 0702 (für den Transport der polaren Phase) und andererseits eine Zahnradpumpe Modell Gather P15133 (für den Transport der ethanolischen Lipidphase). Alternativ wurde für den Transport der ethanolischen Lipidphase auch die pumpenfreie Drucküberlagerung mit Stickstoff angewandt.
a) Aufnehmen einer Druck/Fluss-Kurve für die Ethanol-Phase In der nachfolgenden Tabelle 9 sind die für die Zahnradpumpe ermittelten Werte aufgelistet und in Fig. 12 graphisch dargestellt.
Gemessen wurde der Fluss (Volumensstrom) von Ethanol (92 Vol.%) bei einer Temperatur von 45 - 55 °C durch die Zufuhrleitung für die Lipidphase bzw. durch die anschliessende Bohrung im Kreuzstrommodul. Als Zufuhrleitung von der Pumpe bis hin zum Kreuzstrommodul wurde ein Silikonschlauch mit 1.6 mm Innendurchmesser eingesetzt. Es wurden drei Kreuzstrommodule mit jeweils einer einzigen Bohrung verwendet, wobei die Bohrungen Nennweiten von 150 µm, 200 µm oder 250 µm aufwiesen.
Nachdem es sich gezeigt hatte, dass mittels Drucküberlagerung des Zwischenbehälters (gemäss Fig. 2 und Fig. 3) unter Verwendung von Pressluft oder Stickstoffgas die lipidhältige Phase mindestens ebenso genau und gleichmässig in das Kreuzstrommodul gefördert werden kann, wurde diese pumpenfreie Methode für das erfindungsgemässe Herstellungsverfahren als einfache und wartungsfreie Alternative zur Förderung mittels Zahnradpumpe ebenso verwendet.
b) Aufnehmen einer Flußkurve für die polare Phase Als polare Phase wurde PBS eingesetzt. Die verwendete Pumpe war eine Schlauchquetschpumpe vom Typ Ismatec SA 0702, als Zufuhrleitung von der Pumpe bis zum Kreuzstrommodul wurde ein Silikonschlauch eingesetzt. Drei verschiedene Schlauchdurchmesser wurden getestet: 6, 8 und 10 mm Innendurchmesser. Die Schläuche waren blasenfrei gefüllt. Die Angaben zur Pumpeneinstellung beziehen sich auf die an der Pumpe vorhandene Skalierung zur Wahl der Pumpleistung. Die ermittelten Werte sind nachfolgend in Tabelle 10 sowie in Fig. 13 dargestellt

**Tabelle 9 :**

| Druck/Fluss-Kurve für ethanolische Phase | | | |
|---|---|---|---|
| Fluss [ml/min] | Dosierdruck [bar] bei einem Bohrungsdurchmesser von | | |
| | 150 µm | 200 µm | 250 µm |
| 0.00 | 0.00 | 0.00 | 0.00 |
| 8.50 | 0.10 | | |
| 10.00 | | 0.05 | |
| 12.00 | 0.35 | | |
| 15.00 | | 0.10 | |
| 17.00 | 0.65 | | |
| 20.50 | 1.00 | | |
| 22.00 | | 0.25 | |
| 23.50 | 1.30 | | |
| 24.00 | | | 0.15 |
| 26.00 | | 0.35 | |
| 26.50 | 1.60 | | |
| 27.50 | | | 0.20 |
| 28.50 | | 0.40 | |
| 29.50 | 1.90 | | |
| 31.00 | | 0.50 | 0.25 |
| 32.50 | 2.20 | | |
| 33.00 | | 0.60 | |
| 34.00 | 2.55 | | |
| 35.00 | | | 0.35 |
| 36.00 | 2.90 | 0.70 | |
| 39.00 | | 0.80 | 0.40 |
| 41.00 | | 0.90 | |
| 43.00 | | | 0.50 |
| 46.00 | | 1.10 | 0.55 |
| 49.00 | | 1.25 | |
| 52.00 | | 1.45 | |
| 52.50 | | | 0.75 |
| 57.50 | | | 0.90 |
| 63.00 | | | 1.10 |
| 67.00 | | | 1.20 |
| 72.00 | | | 1.4 |
| 76.00 | | | 1.55 |
| 80.00 | | | 1.7 |
| 85.00 | | | 1.9 |
| 89.00 | | | 2.05 |
| 94.00 | | | 2.2 |
| 99.00 | | | 2.4 |

**Tabelle 10:**

| Eichkurve für PBS-Flüsse mittels Schlauchquetschpumpe | | | |
|---|---|---|---|
| | Fluss [ml/min] | | |
| Pumpeneinstellung | 6 mm ID* | 8 mm ID* | 10 mm ID* |
| 0 | 0 | 0 | 0 |
| 100 | 100 | 170 | 250 |
| 200 | 200 | 360 | 470 |
| 300 | 310 | 550 | 740 |
| 400 | 415 | 710 | 980 |
| 500 | 520 | 890 | 1220 |
| 600 | 625 | 1120 | 1460 |
| 700 | 730 | 1300 | 1800 |
| 800 | 840 | 1510 | 2040 |
| 900 | 950 | 1700 | 2320 |
| 1000 | 1060 | | 2600 |

| | | | |
|---|---|---|---|
| * ID=Innendurchmesser | | | |

## Patentansprüche

1. Vorrichtung zur Herstellung von Lipidvesikeln, welche folgende Bestandteile enthält: eine im Inneren hohle Leitung (1) für den Transport einer polaren Flüssigkeitsphase, eine im Inneren hohle Leitung (2) für den Transport einer lipidhältigen organischen Flüssigkeitsphase, einen Sammelbehälter (7) zur Aufnahme erzeugter Lipidvesikel, sowie Mittel zur Förderung der Flüssigkeitsphasen durch die Leitungen (1) und (2),
**dadurch gekennzeichnet, dass** die Leitung (1) mit der Leitung (2) über eine gemeinsame, flüssigkeitsdurchlässige Öffnung (3) verbunden ist, wobei die gemeinsame Öffnung (3) so angeordnet ist, dass die organische Flüssigkeitsphase aus der Leitung (2) im wesentlichen senkrecht zur Fliessrichtung der in Leitung (1) fliessenden polaren Flüssigkeitsphase durch die Öffnung (3) hindurchtreten und, vorzugsweise in Form eines Sprühnebels, in die an der Öffnung (3) vorbeiströmende polare Flüssigkeitsphase eindringen kann und wobei ausserdem die Leitungen (1) und (2) im Bereich der gemeinsamen Öffnung (3) frei von Turbulenzen oder Scherkräfte erzeugenden Einrichtungen sind.

2. Vorrichtung nach Anspruch 1, **dadurch gekennzeichnet, dass** die Leitungen (1) und (2) im Bereich der gemeinsamen Öffnung (3) frei von Turbulenzen oder Scherkräfte erzeugenden Hindernissen, Rühr- oder Dispergierhilfen sind.

3. Vorrichtung nach Anspruch 1 oder 2, **dadurch gekennzeichnet, dass** der Durchmesser der Öffnung (3) kleiner ist als der Innendurchmesser der Leitungen (1) und (2), und vorzugsweise im Bereich von 50 - 1500 µm liegt.

4. Vorrichtung nach einem der Ansprüche 1 bis 3, **dadurch gekennzeichnet, dass** die Leitungen (1) und (2) im Bereich der gemeinsamen Öffnung (3) entweder parallel oder einander überkreuzend angeordnet sind, oder dass die Leitung (2) mit einem Ende stirnseitig, vorzugsweise T-förmig, an die Aussenwand der Leitung (1) angrenzt.

5. Vorrichtung nach einem der vorhergehenden Ansprüche, **dadurch gekennzeichnet, dass** die Leitungen (1) und (2) zumindest im Bereich der gemeinsamen Öffnung (3) aus chemisch und mechanisch widerstandsfähigem Material, insbesondere aus Edelstahl oder einem geeigneten Hartkunststoff, gefertigt sind.

6. Vorrichtung nach einem der vorhergehenden Ansprüche, **dadurch gekennzeichnet, dass** die Leitungen (1) und (2) im Bereich der gemeinsamen Öffnung (3) als vorgefertigte Einheit, insbesondere als Kreuzstrommodul (4), ausgebildet sind.

7. Vorrichtung nach einem der vorhergehenden Ansprüche, **dadurch gekennzeichnet, dass** stromabwärts von der Öffnung (3) eine Leitung (1') von der Leitung (1) abzweigt und die Leitung (1) im Bereich der Abzweigung oder stromabwärts davon eine regelbare Sperre (15) enthält, mit deren Hilfe zumindest ein Teil des Flüssigkeitsstromes von der Leitung (1) in die Leitung (1') umgelenkt werden kann.

8. Vorrichtung nach einem der vorhergehenden Ansprüche, **dadurch gekennzeichnet, dass** die Mittel zur Förderung der Flüssigkeitsphasen eine Pumpe (6) umfassen, die in der Leitung (1) zwischen Vorratsbehälter (5) und Öffnung (3) angeordnet ist und die polare Flüssigkeitsphase aus dem Vorratsbehälter (5) heraus durch die Leitung (1) in Richtung zur Kontaktfläche mit der Öffnung (3) und weiter in den Sammelbehälter (7) und/oder über eine Leitung (1') zumindest teilweise wieder in den Vorratsbehälter (5) zurück befördert.

9. Vorrichtung nach einem der vorhergehenden Ansprüche, **dadurch gekennzeichnet, dass** die Mittel zur Förderung der Flüssigkeitsphasen eine Pumpe (9) umfassen, mit deren Hilfe die lipidhältige Phase aus einem Vorratsbehälter (8), gegebenenfalls über ein Filter (10), in einen Zwischenbehälter (11), sowie gegebenenfalls weiter durch die Leitung (2) und die Öffnung (3) hindurch in die Leitung (1) befördert wird.

10. Vorrichtung nach einem der Ansprüche 1 bis 8, **dadurch gekennzeichnet, dass** die Mittel zur Förderung der Flüssigkeitsphasen eine Druckquelle (13) umfassen, die mit einem Zwischenbehälter (11), gegebenenfalls über ein zwischengeschaltetes Filter (12), verbunden ist und welche mittels Drucküberlagerung durch Druckluft oder ein unter Druck stehendes Inertgas eine pumpenfreie Förderung der lipidhältigen Phase vom Zwischenbehälter (11) über die Leitung (2) durch die Öffnung (3) hindurch ermöglicht.

11. Vorrichtung nach einem der vorhergehenden Ansprüche, **dadurch gekennzeichnet, dass** sie zwei oder mehr Leitungen (1) und/oder zwei oder mehr Leitungen (2) umfasst, die zwei oder mehr gemeinsame Öffnungen (3) aufweisen.

12. Verfahren zur Herstellung von Lipidvesikeln mit steuerbarer Grössenverteilung durch druckgesteuertes Dosieren einer lipidhältigen Flüssigkeitsphase in eine strömende polare Flüssigkeitsphase unter Verwendung einer Vorrichtung gemäss einem der Ansprüche 1 - 11, wobei die polare Flüssigkeitsphase durch eine Leitung befördert wird, welche zumindest an einer Stelle ihrer Seitenwand eine Öffnung enthält, durch welche die lipidhältige Flüssigkeitsphase unter Druck und in im wesentlichen senkrechter Richtung zur Fliessrichtung der polaren Flüssigkeitsphase gepresst und in die an der Öffnung vorbeiströmende polare Flüssigkeitsphase dosiert, insbesondere gesprüht, wird, wobei sich - spontan und ohne Einwirkung mechanischer Rühr- oder Dispergierhilfen - durch einen kontrolliert steuerbaren "self assembly" Effekt Lipidvesikel mit enger Grössenverteilung bilden.

13. Verfahren nach Anspruch 12, worin die polare Flüssigkeitsphase vor Erreichen der Seitenwandöffnung eine laminare oder annähernd laminare Strömung aufweist.

14. Verfahren nach Anspruch 12 oder 13, worin die lipidhältige Flüssigkeitsphase mit einem Druck von 0.1 bis 15, vorzugsweise 0.3 bis 5 bar, durch die Öffnung hindurch in die vorbeiströmende polare Flüssigkeitsphase dosiert wird.

15. Verfahren nach einem der Ansprüche 12 bis 14, worin die lipidhältige Flüssigkeitsphase in einer Menge von 1 -100 µmol, vorzugsweise 2.5 - 25 µmol Lipid pro 1 ml polarer Flüssigkeitsphase, bezogen auf die Gesamtmenge an eingesetzter polarer Phase, zudosiert wird.

16. Verfahren nach Anspruch 15, worin nur ein Teil, vorzugsweise 10 - 50 Vol.%, der gesamten polaren Flüssigkeitsphase durch die Leitung hindurch zur Öffnung und - nach Beladung mit lipidhältiger Flüssigkeitsphase - als konzentrierte Vesikeldispersion weiter zu einem Sammelbehälter befördert wird, während der übrige Teil der polaren Flüssigkeitsphase zur Verdünnung der konzentrierten Vesikeldispersion verwendet und vorzugsweise im Sammelbehälter vorgelegt wird.

17. Verfahren nach einem der Ansprüche 12 bis 16, worin die lipidhältige Flüssigkeitsphase Ethanol als Lösungsmittel enthält und in einer Menge der polaren Flüssigkeitsphase zudosiert wird, die eine Endkonzentration von maximal 10 Vol. %, vorzugsweise maximal 7.5 Vol.% Ethanol in der polaren Flüssigkeitsphase ergibt.

18. Verfahren nach einem der Ansprüche 12 bis 17, worin zumindest ein Teil der polaren Flüssigkeitsphase nach Beladung mit lipidhältiger Flüssigkeitsphase rezirkuliert und einer neuerlichen Beladung mit lipidhältiger Flüssigkeitsphase zugeführt wird.

19. Verfahren nach einem der Ansprüche 12 bis 18, worin die polare Flüssigkeitsphase und/oder die lipidhältige Flüssigkeitsphase zumindest eine gewünschte Substanz, insbesondere einen pharmazeutischen Wirkstoff, zur Beladung der Lipidvesikel enthält.

20. Verfahren nach einem der Ansprüche 12 bis 19, worin zumindest 60 % der entstehenden Lipidvesikeln einen Durchmesser von 100 bis 350 nm aufweisen.

## Claims

1. Device for producing lipid vesicles, which comprises the following components: a pipe (1) which is hollow in the interior and is intended for transporting a polar liquid phase, a pipe (2) which is hollow in the interior and is intended for transporting a lipid-containing organic liquid phase, a collecting container (7) for receiving lipid vesicles produced, and means for transporting the liquid phases through the pipes (1) and (2), **characterized in that** the pipe (1) is connected to the pipe (2) via a common, liquid-permeable orifice (3), the common orifice (3) being arranged so that the organic liquid phase from the pipe (2) can pass through the orifice (3) substantially perpendicularly to the direction of flow of the polar liquid phase flowing in pipe (1) and can enter, preferably in the form of a spray mist, the polar liquid phase flowing past the orifice (3), and furthermore the pipes (1) and (2) in the region of the common orifice (3) being free of means generating turbulences or shear forces.

2. Device according to Claim 1, **characterized in that** the pipes (1) and (2) in the region of the common orifice (3) are free of obstacles or stirring or dispersing aids generating turbulences or shear forces.

3. Device according to Claim 1 or 2, **characterized in that** the diameter of the orifice (3) is smaller than the internal diameter of the pipes (1) and (2) and is preferably in the range of 50 - 1500 µm.

4. Device according to any of Claims 1 to 3, **characterized in that** the pipes (1) and (2) are arranged either parallel or intersecting one another in the region of the common orifice (3), or that the pipe (2) is adjacent at one end face, preferably in a T-shaped manner, to the outer wall of the pipe (1).

5. Device according to any of the preceding Claims, **characterized in that** the pipes (1) and (2) are produced from chemically and mechanically resistant material, in particular from stainless steel or a suitable rigid plastic, at least in the region of the common orifice (3).

6. Device according to any of the preceding Claims, **characterized in that** the pipes (1) and (2) are formed as a prefabricated unit in the region of the common orifice (3), in particular as cross-flow module (4).

7. Device according to any of the preceding Claims, **characterized in that** a pipe (1') branches out from the pipe (1) downstream of the orifice (3) and, in the region of the branch or downstream thereof, the pipe (1) contains a controllable gate (15) with the aid of which at least a part of the liquid stream can be deflected from the pipe (1) into the pipe (1').

8. Device according to any of the preceding Claims, **characterized in that** the means for transporting the liquid phases comprise a pump (6) which is arranged in the pipe (1) between storage container (5) and orifice (3) and transports the polar liquid phase out of the storage container (5) through the pipe (1) in the direction of the contact area with the orifice (3) and further into the collecting container (7) and/or via a pipe (1') at least partly back into the storage container (5).

9. Device according to any of the preceding Claims, **characterized in that** the means for transporting the liquid phases comprise a pump (9) with the aid of which the lipid-containing phase is transported from a storage container (8), optionally via a filter (10), into an intermediate container (11), and optionally further through the pipe (2) and the orifice (3) into the pipe (1).

10. Device according to any of Claims 1 to 8, **characterized in that** the means for transporting the liquid phases comprise a pressure source (13) which is connected to an intermediate container (11), optionally via an intermediate filter (12), and which permits pump-free transport of the lipid-containing phase from the intermediate container (11) via the pipe (2) through the orifice (3) by means of pressure superposition by compressed air or an inert gas under pressure.

11. Device according to any of the preceding Claims, **characterized in that** it comprises two or more pipes (1) and/or two or more pipes (2) which have two or more common orifices (3).

12. Method for producing lipid vesicles having a controllable size distribution by pressure-controlled metering of a lipid-containing liquid phase into a flowing polar liquid phase with the use of a device according to any of Claims 1 - 11, the polar liquid phase being transported through a pipe which contains, at least at one point of its side wall, an orifice through which the lipid-containing liquid phase is forced under pressure and in a direction substantially perpendicular to the direction of flow of the polar liquid phase and is metered, in particular sprayed, into the polar liquid phase flowing past the orifice, lipid vesicles having a narrow size distribution forming - spontaneously and without the action of mechanical stirring or dispersing aids - by a controllable self-assembly effect.

13. Method according to Claim 12, in which the polar liquid phase has laminar or virtually laminar flow before reaching the side wall orifice.

14. Method according to Claim 12 or 13, in which the lipid-containing liquid phase is metered at a pressure of 0.1 to 15, preferably 0.3 to 5, bar through the orifice into the polar liquid phase flowing past.

15. Method according to any of Claims 12 to 14, in which the lipid-containing liquid phase is metered in an amount of 1 -100 µmol, preferably 2.5 - 25 µmol, of lipid per 1 ml of polar liquid phase, based on the total amount of polar phase used.

16. Method according to Claim 15, in which only a part, preferably 10 - 50% by volume, of the total polar liquid phase is transported through the pipe to the orifice and - after loading with lipid-containing liquid phase - is transported further as concentrated vesicle dispersion to a collecting container, while the remaining part of the polar liquid phase is used for diluting the concentrated vesicle dispersion and is preferably initially introduced into the collecting container.

17. Method according to any of Claims 12 to 16, in which the lipid-containing liquid phase contains ethanol as a solvent and is metered into the polar liquid phase in an amount which gives a final concentration of not more than 10% by volume, preferably not more than 7.5% by volume, of ethanol in the polar liquid phase.

18. Method according to any of Claims 12 to 17, in which at least a part of the polar liquid phase is recirculated after loading with lipid-containing liquid phase and is fed for further loading with lipid-containing liquid phase.

19. Method according to any of Claims 12 to 18, in which the polar liquid phase and/or the lipid-containing liquid phase contains at least one desired substance, in particular a pharmaceutical active substance, for loading the lipid vesicles.

20. Method according to any of Claims 12 to 19, in which at least 60% of the lipid vesicles forming have a diameter of 100 to 350 nm.

## Revendications

1. Dispositif pour produire des vésicules lipidiques qui comprend les éléments suivants : une conduite (2) creuse à l'intérieur pour le transport d'une phase liquide polaire, une conduite (2) creuse à l'intérieur pour le transport d'une phase liquide organique lipidique, un récipient collecteur (7) pour recueillir les vésicules lipidiques produites, ainsi que des moyens assurant l'alimentation en phases liquides par l'intermédiaire des conduites (1) et (2),
**caractérisé en ce que** la conduite (1) est en communication fluide avec la conduite (2) par l'intermédiaire d'une ouverture commune (3), où l'ouverture commune (3) est agencée de manière à ce que la phase liquide organique passe de la conduite (2) par l'ouverture (3) suivant une direction sensiblement perpendiculaire à la direction du flux de la phase liquide polaire circulant dans la conduite (1), de préférence sous la forme d'un brouillard de fines gouttelettes dans lequel la phase liquide polaire circulant au droit de l'ouverture (3) peut pénétrer et où, en outre, les conduites (1) et (2) sont exemptes, dans la région de l'ouverture commune (3), de composants susceptibles de créer des turbulences ou des forces de cisaillement.

2. Dispositif selon la revendication 1, **caractérisé en ce que** les conduites (1) et (2) sont exemptes, dans la région de l'ouverture commune (3), d'obstacles ou de moyens favorisant l'agitation ou la dispersion, susceptibles de créer des turbulences ou des forces de cisaillement.

3. Dispositif selon la revendication 1 ou la revendication 2, **caractérisé en ce que** le diamètre de l'ouverture (3) est plus petit que le diamètre interne des conduites (1) et (2) et se situe, de préférence, dans la plage allant de 50 à 1500 µm.

4. Dispositif selon l'une des revendications 1 à 3, **caractérisé en ce que** les conduites (1) et (2) sont disposées dans la région de l'ouverture commune (3) soit de manière parallèle, soit de manière à se croiser mutuellement, soit encore de manière à ce que la conduite (2) vienne par son extrémité frontale contre la paroi externe de la conduite (1), de préférence en formant un T.

5. Dispositif selon l'une des revendications précédentes, **caractérisé en ce que** les conduites (1) et (2) sont réalisées, du moins dans la région de l'ouverture commune (3), en un matériau chimiquement et mécaniquement résistant, en particulier en un acier spécial ou en un matériau synthétique dur approprié.

6. Dispositif selon l'une des revendications précédentes, **caractérisé en ce que** les conduites (1) et (2) sont réalisées, dans la région de l'ouverture commune (3), sous la forme d'une unité préfabriquée, en particulier d'un module (4) à flux croisés.

7. Dispositif selon l'une des revendications précédentes, **caractérisé en ce qu'**en aval de l'ouverture (3), une conduite (1') est montée en dérivation sur la conduite (1) et **en ce que** la conduite (1) comporte, dans la région de la dérivation ou en aval de celle-ci, une soupape ajustable (15), grâce à laquelle au moins une partie du flux du liquide peut être dévié de la conduite (1) vers la conduite (1').

8. Dispositif selon l'une des revendications précédentes, **caractérisé en ce que** les moyens assurant l'alimentation en phases liquides comprennent une pompe (6) qui est disposée sur la conduite (1) entre le réservoir de stockage (5) et l'ouverture (3) et qui achemine la phase liquide polaire du réservoir de stockage (5) par la conduite (1) dans la direction de la surface de contact comportant l'ouverture (3) et ensuite vers le récipient collecteur (7) et / ou la retourne, au moins partiellement, par la conduite (1') vers le réservoir de stockage (5).

9. Dispositif selon l'une des revendications précédentes, **caractérisé en ce que** les moyens assurant l'alimentation en phases liquides comprennent une pompe (9), grâce à laquelle la phase lipidique est acheminée depuis un réservoir de stockage (8) - le cas échéant via un filtre (10) - vers un réservoir intermédiaire. (11) et ensuite, par la conduite (2) et l'ouverture (3), vers la conduite (1).

10. Dispositif selon l'une des revendications 1 à 8, **caractérisé en ce que** les moyens assurant l'alimentation en phase liquide comprennent une source de pression (13) qui est en communication - le cas échéant via un filtre intermédiaire (12) avec le réservoir intermédiaire (11) et qui assure - grâce à l'application d'une pression fournie par de l'air sous pression ou par un gaz inerte sous pression une alimentation sans pompe en phase lipidique depuis le réservoir intermédiaire (11) par la conduite (2) et par l'ouverture (3).

11. Dispositif selon l'une des revendications précédentes, **caractérisé en ce qu'**il comprend deux conduites (1) ou davantage et / ou deux conduites (2) ou davantage, qui présentent deux ouvertures communes (3) ou davantage.

12. Procédé pour produire des vésicules lipidiques avec une répartition de la taille susceptible d'être ajustée par un contrôle de la pression de l'introduction d'une phase liquide lipidique dans un flux d'une phase liquide polaire, mis en oeuvre avec le dispositif selon l'une des revendications 1 - 11, dans lequel la phase liquide polaire est acheminée par une conduite qui comprend, au moins en un emplacement de sa paroi latérale, une ouverture par laquelle la phase liquide lipidique sous pression est poussée suivant une direction sensiblement perpendiculaire au flux du liquide polaire pour être introduite et, plus particulièrement pulvérisée dans le flux de la phase liquide polaire circulant au droit de l'ouverture, ce qui permet de produire de manière spontanée et sans l'intervention de moyens mécaniques favorisant l'agitation ou la dispersion, par un effet « d'auto-assemblage » contrôlé de manière ajustable, des vésicules lipidiques avec une répartition des tailles qui est étroite.

13. Procédé selon la revendication 12, dans lequel la phase liquide polaire présente un écoulement laminaire ou presque laminaire avant son arrivée à l'ouverture dans la paroi latérale.

14. Procédé selon la revendication 12 ou la revendication 13, dans lequel la phase liquide lipidique est introduite par l'ouverture, dans le flux de la phase liquide polaire sous une pression de 0,1 à 15 et, de préférence, de 0,3 à 5 bars.

15. Procédé selon l'une des revendications 12 à 14, dans lequel la phase liquide lipidique est introduite en une quantité correspondant à 1 - 100 µmoles et, de préférence à 2,5 - 25 µmoles de lipide par 1 ml de la phase liquide polaire totale mise en oeuvre.

16. Procédé selon la revendication 15, dans lequel seulement une partie, de préférence de 10 - 50 % en volume de la phase liquide polaire totale, est acheminée par la conduite vers l'ouverture et ensuite - après avoir été chargée avec la phase liquide lipidique pour former une dispersion concentrée de vésicules - vers un récipient collecteur, pendant que la partie restante de la phase liquide polaire est utilisée pour diluer la dispersion concentrée de vésicules en étant, de préférence, placée au préalable dans le récipient collecteur.

17. Procédé selon l'une des revendications 12 à 16, dans lequel la phase liquide lipidique contient de l'éthanol en tant que milieu solvant, la quantité d'éthanol ajoutée à la phase liquide polaire étant telle que sa concentration finale dans la phase liquide polaire soit au maximum de 10 % en volume et, de préférence, au maximum de 7,5 % en volume.

18. Procédé selon l'une des revendications 12 à 17, dans lequel au moins une partie de la phase liquide polaire est soumise à une recirculation après avoir été chargée avec la phase liquide lipidique, pour être à nouveau chargée avec la phase liquide lipidique.

19. Procédé selon l'une des revendications 12 à 18, dans lequel la phase liquide polaire ou / et la phase liquide lipidique contient / contiennent au moins une substance souhaitée, en particulier une substance active pharmaceutique, pour charger les vésicules lipidiques.

20. Procédé selon l'une des revendications 12 à 19, dans lequel au moins 60 % des vésicules lipidiques formées présentent un diamètre de 100 à 350 nm.
